(19) Europäisches Patentamt
European Patent Office
Office européen des brevets

(11) Numéro de publication: **0 287 465 B1**

(12) # FASCICULE DE BREVET EUROPEEN

(45) Date de publication de fascicule du brevet: **27.01.93**

(51) Int. Cl.[5]: **C07D 257/02**, C07D 259/00, A61K 49/00, C07F 5/00, C07F 15/02, C07F 13/00

(21) Numéro de dépôt: **88400895.4**

(22) Date de dépôt: **13.04.88**

Le dossier contient des informations techniques présentées postérieurement au dépôt de la demande et ne figurant pas dans le présent fascicule.

(54) **Ligands cycliques azotés, complexes métalliques formés par ces ligands, compositions de diagnostic contenant ces complexes et procédé de préparation des ligands.**

(30) Priorité: **14.04.87 FR 8705288**

(43) Date de publication de la demande: **19.10.88 Bulletin 88/42**

(45) Mention de la délivrance du brevet: **27.01.93 Bulletin 93/04**

(84) Etats contractants désignés: **AT BE CH DE ES FR GB GR IT LI LU NL SE**

(56) Documents cités:
**EP-A- 0 124 766**
**EP-A- 0 255 471**
**WO-A-82/04252**
**WO-A-86/02352**
**FR-A- 2 539 996**

**CHEMICAL ABSTRACTS, vol. 97, no. 24, 13 décembre 1982, page 617, résumé no. 206960z, Columbus, Ohio, US; C.C. BRYDEN et al.: "Multinuclear NMR study of three aqueous lanthanide shift reagents: complexes with EDTA and two macrocyclic ligands"**

(73) Titulaire: **GUERBET S.A.**
**15, rue des Vanesses Z.A.C. Paris Nord II**
**F-93420 Villepinte(FR)**

(72) Inventeur: **Schaefer Michel**
**4 Rue François-Girardon**
**F-91380 Chilly-Mazarin(FR)**
Inventeur: **Doucet Didier**
**141, rue du Docteur David Rosenseld**
**F-93230 Romainville(FR)**
Inventeur: **Bonnemain Bruno**
**58 rue du Maréchal Joffre**
**F-77270 Villeparisis(FR)**
Inventeur: **Meyer Dominique**
**23 rue du Tage**
**F-75013 Paris(FR)**

(74) Mandataire: **Le Guen, Gérard et al**
**CABINET LAVOIX 2, place d'Estienne d'Orves**
**F-75441 Paris Cédex 09(FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services

## Description

La présente invention concerne de nouveaux ligands cycliques azotés et des complexes métalliques formés par ces ligands, les applications de ces complexes en imagerie par résonance magnétique, en radiologie par rayons X et comme agents de déplacement chimique in vivo.

L'invention concerne également un procédé de préparation de ces ligands.

EP-A-0 124 766 décrit un agent de contraste pour IRM formé d'un composé paramagnétique et, notamment d'une polyamine.

FR-A-2 539 996 décrit un agent de diagnostic formé par l'anion d'un acide complexant et par un ou plusieurs ions centraux d'éléments de numéros atomiques 21 à 29, 42, 44 ou 57 à 83.

WO-A-8 602 352 décrit des chélates de gadolinium avec un composé choisi parmi le DOTRA, le DOTA et le NOTA.

Cependant aucun document ne décrit des ligands cycliques azotés comportant un substituant sur un atome de carbone du macrocycle.

L'invention a ainsi pour objet des ligands de formule

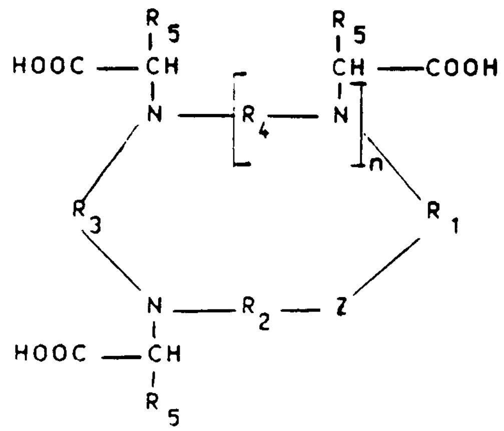

I

dans laquelle

$R_1$ représente un radical de formule

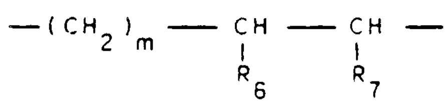

$R_6$ étant choisi parmi un groupe alkyle en $C_1$-$C_{14}$, un groupe hydroxyalkyle en $C_1$-$C_4$ et un groupe polyhydroxyalkyle en $C_1$-$C_4$ et un groupe de formule

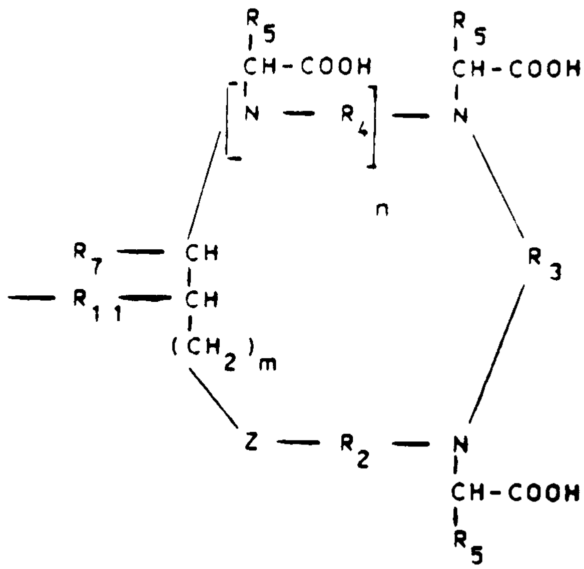

$R_{11}$ étant choisi parmi les groupes A et les groupes de formule $-(CH_2)_t$ - Y - A - Y-$(CH_2)_t-$
A étant choisi parmi les groupes alkylène en $C_1$-$C_8$, hydroxyalkylène en $C_1$-$C_8$ et polyhydroxyalkylène en $C_1$ - $C_8$,
Y étant choisi parmi

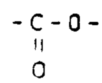

et -O- et t = 1 à 4,
$R_7$ étant choisi parmi un atome d'hydrogène, un groupe alkyle en $C_1$-$C_{14}$, un groupe hydroxyalkyle en $C_1$-$C_4$ et un groupe polyhydroxyalkyle en $C_1$-$C_4$,
m = 0 ou 1
$R_2$,$R_3$,$R_4$ identiques ou différents représentent un radical de formule

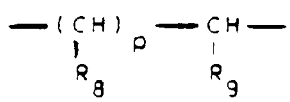

$R_8$ et $R_9$ identiques ou différents étant choisis parmi un atome d'hydrogène, un groupe alkyle en $C_1$-$C_{14}$, un groupe hydroxyalkyle en $C_1$-$C_4$, et un groupe polyhydroxyalkyle en $C_1$-$C_4$,
p = 1 ou 2
n = 0,1 ou 2 et
$R_5$ est choisi parmi un atome d'hydrogène, un groupe alkyle en $C_1$-$C_4$, un groupe hydroxyalkyle en $C_1$-$C_4$ et un groupe polyhydroxyalkyle en $C_1$-$C_4$, et
Z est choisi parmi un atome d'oxygène et un groupe de formule

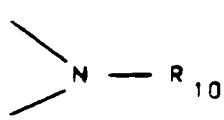

$R_{10}$ étant choisi parmi un atome d'hydrogène, un groupe alkyle en $C_1$-$C_{14}$, un groupe hydroxyalkyle en $C_1$-$C_4$, un groupe polyhydroxyalkyle en $C_1$-$C_4$, un goupe de formule

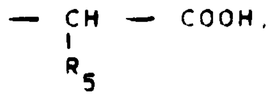

$R_5$ ayant la signification donnée précédemment, et un groupe de formule

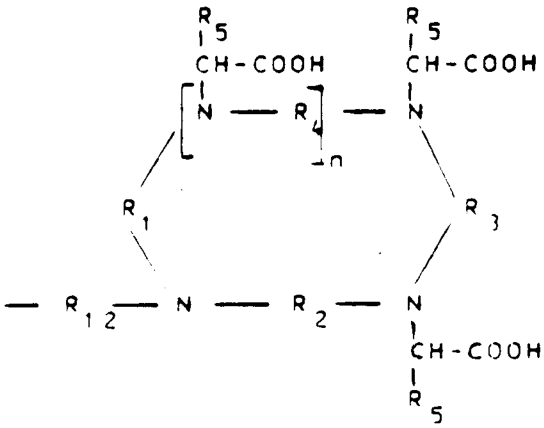

$R_{12}$ étant choisi parmi les groupes alkylène en $C_1$-$C_8$, hydroxyalkylène en $C_1$-$C_8$ et polyhydroxyalkylène en $C_1$ - $C_8$, ainsi que leurs sels.

Les ligands de formule I peuvent être préparés par réaction d'un composé de formule

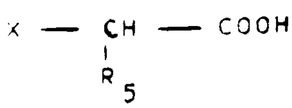

II

dans laquelle $R_5$ à la signification donnée ci-dessus et X représente un groupe labile tel qu'un atome de chlore, de brome ou d'iode ou un groupe tosyloxy ou mésyloxy, avec une amine cyclique de formule

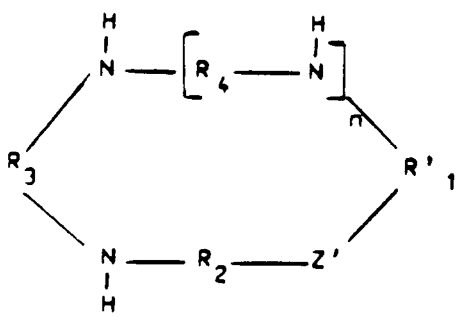

III

dans laquelle $R_2$, $R_3$, $R_4$, et n ont la signification donnée ci-dessus,
$R'_1$ représente un radical de formule

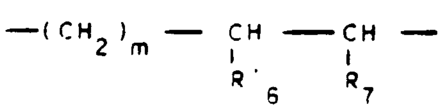

$R'_6$ étant choisi parmi un groupe alkyle en $C_1$-$C_{14}$ , un groupe hydroxyalkyle en $C_1$-$C_4$, un groupe polyhydroxyalkyle en $C_1$-$C_4$ et un groupe de formule

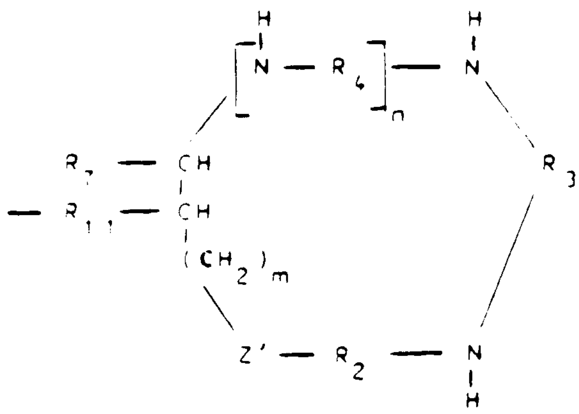

$R_2$, $R_3$, $R_4$, $R_7$, $R_{11}$, m, n, ayant la signification donnée ci-dessus, et Z′ est choisi parmi un atome d'oxygène et un groupe de formule

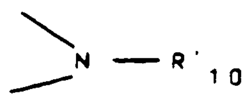

$R'_{10}$ étant choisi parmi un atome d'hydrogène, un groupe alkyle en $C_1$-$C_{14}$, un groupe hydroxyalkyle en $C_1$-$C_4$, un groupe polyhydroxyalkyle en $C_1$-$C_4$, et un groupe de formule

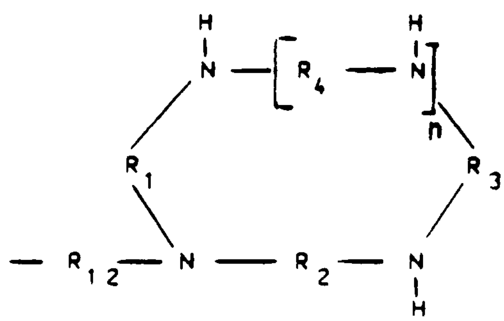

$R_1$, $R_2$, $R_3$, $R_4$, $R_{12}$ et n ayant la signification donnée ci-dessus.

Les ligands de formule I peuvent être également préparés selon une réaction de Strecker, par réaction sur une amine cyclique de formule III d'un aldéhyde de formule,

$R_5$-CHO IIa

dans laquelle $R_5$ a la signification donnée précédemment, en présence d'acide cyanhydrique ou plus généralement d'ions cyanure (KCN, NaCN).

Les composés de formule III dans laquelle Z′ est un groupe

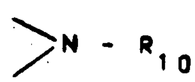

peuvent être préparés

a) par réaction d'une polyamine de formule

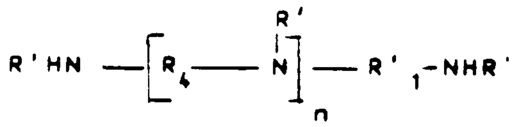 IV

dans laquelle n,$R'_1$ et $R_4$ ont la signification donnée précédemment et R′ représente un groupe tosyle, mésyle ou benzène sulfonyle
avec un composé de formule

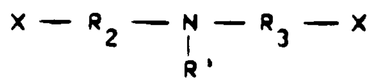 V

dans laquelle $R_2$, $R_3$ et R′ ont la signification donnée précédemment et X représente un groupe labile tel qu'un groupe tosyloxy, mésyloxy ou un atome de chlore, de brome ou d'iode, ou
b) par réaction d'une diamine de formule

$R'HN—R'_1—NH—R'$ X

dans laquelle $R'_1$ et R′ ont la signification donnée précédemment,
avec un composé de formule

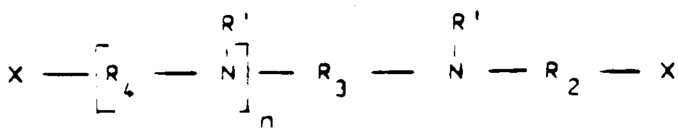 XI

Cette réaction de cyclisation est réalisée avantageusement en présence d'un catalyseur de transfert de phase.

Les polyamines de formule IV peuvent être obtenues à partir de dihydroxylamines selon le schéma suivant :

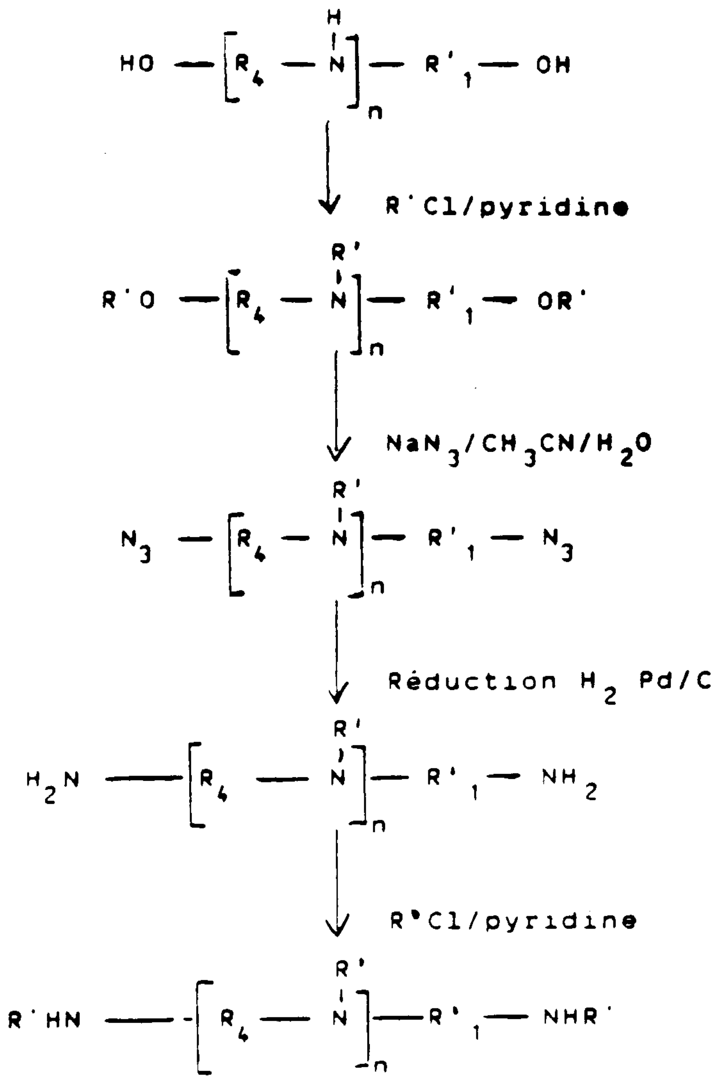


VI

VII

VIII

IX

IV

En variante on peut faire réagir sur les composés de formule VII le phtalimide et effectuer une hydrazinolyse pour passer des composés de formule VII aux composés de formule IX.

Les composés de formule III comportant deux cycles azotés peuvent être préparés selon les procédés précédemment définis.

C'est ainsi que l'on peut faire réagir une polyamine de formule

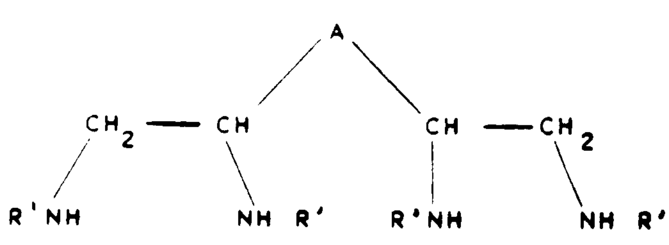

XII

dans laquelle A et R' ont la signification donnée précédemment avec un composé de formule XI pour obtenir un composé de formule III dans laquelle $R_{11}$ est un groupe A.

La polyamine de formule XII peut être obtenue à partir d'un dérivé tétrahalogéné par substitution nucléophile en présence d'azoture de sodium puis réduction en présence d'hydrogène et de palladium sur charbon.

En variante, les composés de formule I comportant deux cycles azotés et dans lesquels $R_{11}$ est un

groupe A, peuvent être préparés par condensation d'un composé de formule

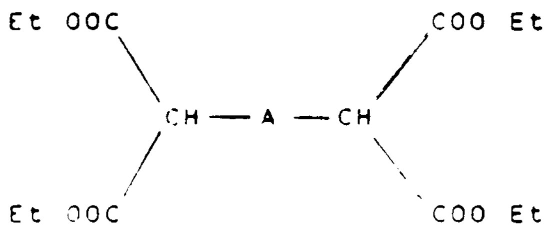

XIII

avec une polyamine de formule

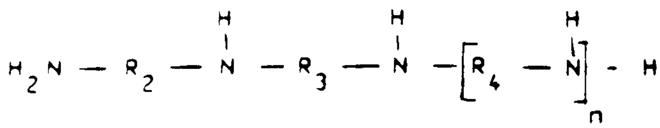

XIV

suivie d'une réduction au diborane, selon un procédé décrit par Tabushi et al. (Tetra Letters 12, 1049, 1977).

Les composés de formule I comportant deux cycles azotés sont ensuite obtenus à partir des composés de formule III à deux cycles comme décrit précédemment.

En variante, des composés de formule I comportant deux cycles azotés peuvent être obtenus par condensation d'un composé de formule I dans laquelle $R_1$ est un radical de formule

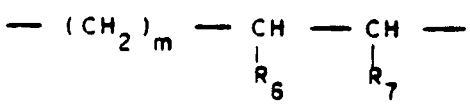

où $R_6$ est un groupe hydroxyalkyle, avec un composé bifonctionnel activable de formule

$X_1—A—X_1$

$X_1$ étant un groupe COOH, COCl ou anhydride d'acide.

Les composés de formule I présentant deux cycles azotés peuvent également être préparés par condensation d'un composé de formule

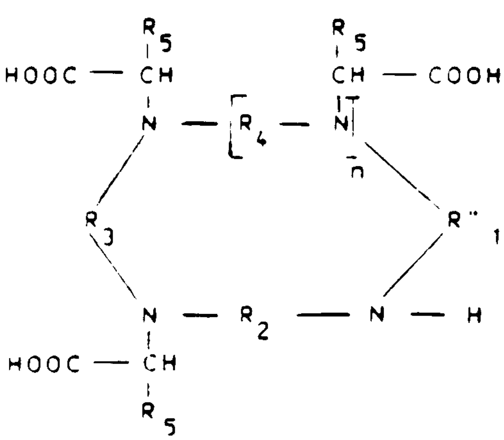

XV

dans laquelle $R''_1$ est un radical de formule

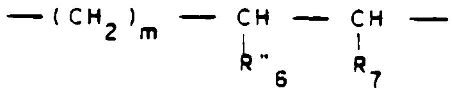

m et $R_7$ ayant la signification donnée précédemment et $R''_6$ étant choisi parmi un principe alkyle en $C_1$-$C_4$, un groupe hydroxyalkyle en $C_1$-$C_4$ et un groupe polyhydroxyalkyle en $C_1$-$C_4$, avec un composé de formule

X—$R'_{12}$—X XVI

X ayant la signification donnée précédemment et $R'_{12}$ représentant un groupe $R_{12}$ éventuellement protégé.

On obtient ainsi des composés de formule

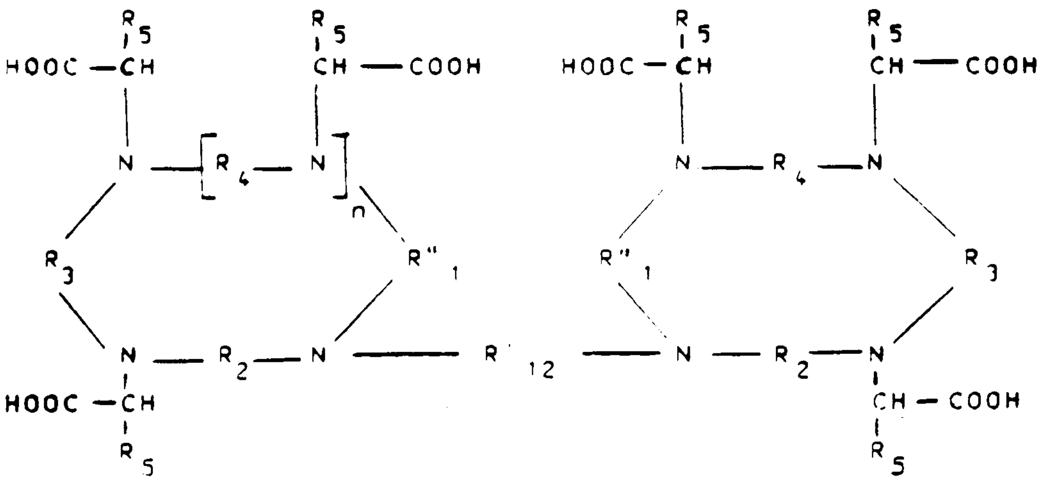

XVII

La présente invention a en outre pour objet des complexes formés par les ligands de formule I avec des ions métalliques choisis parmi les ions des lanthanides (numéros atomiques 57 à 71), les ions des métaux de transition (numéros atomiques 21 à 29), notamment $Mn^{2+}$, $Fe^{3+}$ et $Cr^{3+}$, et les ions des métaux de numéros atomiques 55, 56, 82 et 83, ainsi que les sels de ces complexes avec des bases minérales ou organiques pharmaceutiquement acceptables ou des aminoacides basiques.

Dans ces complexes les ions métalliques sont de préférence le gadolinium, l'europium, le dysprosium, le fer ($Fe^{3+}$) et le manganèse ($Mn^{2+}$).

Comme exemples de sels on peut citer ceux formés avec l'hydroxyde de sodium, la N-methylglucamine, la diéthanolamine, la lysine et l'arginine.

Les complexes peuvent être obtenus par réaction des ligands avec un sel ou un oxyde des métaux dans un solvant aqueux et éventuellement neutralisation pour former un sel.

Il va de soi que la présente invention englobe non seulement les ligands de formule I et les complexes précédemment définis sous forme de mélange racémique mais également les stéréoisomères de ces ligands et complexes.

Les complexes selon l'invention peuvent en outre être liés à une macromolécule qui est susceptible de se fixer préférentiellement sur un organe. C'est ainsi que les complexes selon l'invention peuvent être liés à des protéines et notamment des anticorps.

Ils peuvent en outre être encapsulés notamment dans des liposomes.

Les complexes selon l'invention formés par les ligands de formule I avec des ions paramagnétiques et leurs sels avec des bases pharmaceutiquement acceptables peuvent être utilisés comme agent de contraste en imagerie par résonance magnétique et, comme agent de déplacement chimique in vivo.

Les complexes selon l'invention formés par des ligands de formule I avec des ions des lanthanides (numéros atomiques 57 à 71) ou des ions des métaux de numéro atomique 55, 56, 82 et 83 et leurs sels avec des bases pharmaceutiquement acceptables peuvent être utilisés comme produits de contraste en radiographie par rayons X. A cet effet, on préfère en particulier les complexes formés avec les ions des métaux suivants : Gd, Er, Dy, Tb, Ce, La, Ba et Cs.

La présente invention a en conséquence également pour objet une composition de diagnostic administrable à l'homme caractérisé en ce qu'elle comprend au moins un complexe formé par un ligand de formule I avec des ions métalliques choisis parmi les ions des lanthanides, les ions des métaux de transition et les ions des métaux de numéro atomique 55, 56, 82 et 83, ainsi que les sels de ces complexes avec des bases minérales, ou organiques, pharmaceutiquement acceptables ou des aminoacides basiques.

Ces compositions peuvent être constituées notamment par des solutions dans un solvant aqueux physiologiquement acceptable d'un complexe selon l'invention.

Les compositions de diagnostic selon l'invention peuvent être administrées :

- par voie parentérale dont la voie intraveineuse, la voie intra-artérielle, la voie intralymphatique, la voie sous cutanée
- par voie orale
- par voie sous-arachnoïdienne
- par voie intrabronchique sous forme d'aérosol
- par voie intraarticulaire
- localement pour la visualisation des cavités (par exemple utérus).

En imagerie par résonance magnétique, les doses sont très variables selon les voies d'administration.

Pour la voie intraveineuse ou intra-artérielle, la dose est d'environ 0,01 à 2 mM/kg.

Pour la voie orale cette dose peut aller jusqu'à 10 mM/kg.

Pour les autres voies d'administration, les doses utiles sont généralement inférieures à 1 mM/kg et même généralement pour la voie sous-arachnoïdienne inférieure à 0,05 mM/kg.

Dans l'utilisation comme agents de déplacement chimique in vivo et comme agent de contraste en radiologie par rayons X les doses sont les mêmes, sauf par la voie intraveineuse ou intra-artérielle où les doses peuvent être inférieures ou égales à 5 mM/kg.

En outre, les complexes selon l'invention formés par les ligands de formule I avec des ions radioactifs ainsi que leurs sels avec des bases pharmaceutiquement acceptables peuvent être utilisés comme agents de diagnostic ou de thérapie en médecine nucléaire. Des exemples d'ions radioactifs sont des radioisotopes d'éléments tels que le cuivre, le cobalt, le gallium, le germanium, l'indium et surtout le technétium (Tc 99 m).

Les exemples suivants illustrent la préparation des composés selon la présente demande.

Dans ces exemples :

- Les spectres RMN ont été réalisés sur un appareil Varian EM 360 à 60 MHz avec le TMs comme référence interne. Le solvant est $CDCl_3$ sauf indication contraire.
- Les spectres IR ont été réalisés sur un appareil Perkin-Elmer 1320. Les spectres des solides sont enregistrés sous forme de pastilles de KBr. Dans le cas des liquides (huiles) ils sont enregistrés sans solvant.
- Le terme "tampon" utilisé lors de la chromatographie sur couche mince désigne un mélange $NH_4OH$ 1,5 M et $(NH_4)_2CO_3$ 1,5 M.
- Les points de fusion ont été enregistrés sur banc Kofler.
- Les termes utilisés lors des dosages en cours de complexation :"absence de $Gd^{3+}$ libre et de ligand libre" s'entendent dans les limites de détection des méthodes utilisées c'est-à-dire <4 ppm et <5 ppm pour $Gd^{3+}$ et ligand respectivement.

<u>Exemple 1</u> - <u>Préparation de l'acide 2,6-diméthyl-1,4,7,10-tétraazacyclododécane-N,N′, N″, N‴-tétraacétique</u>

a) Préparation de la N-tosyl-bis(2-tosyloxy propyl)amine.

A une solution de 248 g (1,3 mole) de chlorure de tosyle dans 200 $cm^3$ de pyridine à 0 ° C, est ajoutée une solution de 53,2 g (0,4 mole) de diiso-propanolamine dans 50 $cm^3$ de pyridine goutte à goutte en refroissant de telle sorte que la température soit maintenue entre 0 et 5 ° C. L'ensemble est laissé à cette température pendant 72 h. Le mélange est alors versé sur 2 l. d'eau + glace et 250 $cm^3$ d'acide chlorhydrique concentré.

Le dérivé tosylé est extrait avec 2 l. de chlorure de méthylène. La phase organique est séchée sur sulfate de sodium, filtrée puis décolorée au noir 3 SA et refiltrée sur un lit de silice. Après évaporation du solvant il reste 193,8 g d'une huile jaune (rendement 81%; Rf = 0,7 silice/$CH_2Cl_2$/acétone/98/2) qui est utilisée pour la suite des réactions sans autre purification.

Spectre RMN$^1$H : 6H $CH_3$ (doublet 1,2 et 1,3 ppm); 9H $CH_3$ tosyl (singulet 2,5 ppm); 4H $CH_2$ (multiplet centré à 3,3 ppm); 2H CH (quadruplet entre 4,7 et 5,1 ppm); 12H aromatiques (multiplet 7,3 et 8 ppm).

b) Préparation de la N-tosyl-bis(2-azido propyl)amine

A 193,8 g (0,32 mole) du composé obtenu en a dans 1,2 l d'acétonitrile et 300 $cm^3$ d'eau, sont ajoutés 65,1 g d'azoture de sodium (1 mole). Le mélange est agité et chauffé à 75°C pendant 48h. Après refroissement l'acétonitrile est évaporé sous vide.

Le résidu est repris avec 1l. de chlorure de méthylène. La phase organique est lavée à l'eau, séchée et filtrée sur lit de silice (200 g). Après évaporation il reste 82 g d'huile jaune claire (rendement 75%; Rf = 0,85 silice $CH_2Cl_2$/acétone/92/2) suffisamment pure pour être utilisée directement.

Spectre IR $N_3$ = 2100 $cm^{-1}$ intense.

c) Préparation de la N-tosyl-bis(2-amino propyl)amine

82,2 g (0,244 mole) de composé obtenu en b sont dissous dans 500 $cm^3$ d'éthanol contenant 8 g de charbon palladié à 5% à 50% d'humidité.

L'ensemble est agité vigoureusement alors que l'on fait passer un léger courant d'hydrogène (évacuation de l'azote qui se dégage). Après 8h à température ambiante, la CCM montre l'absence de fonction azide. Le mélange est alors filtré et évaporé. On obtient 68,4 g, une huile jaune claire (rendement 98,5%; Rf = 0,6 silice/MeOH/$NH_4OH$.95/5) qui est utilisé sans autre purification.

Spectre RMN : 6H $CH_3$ (doublet 0,9 et 1 ppm); 3H $CH_3$ tosyl (singulet à 2,4 ppm); 6H $CH_2$ et CH (massif complexe entre 2,7 et 3,2 ppm); 4H aromatiques (multiplet entre 7,1 et 7,7 ppm).

d) Préparation de la N-tosyl-bis/2(tosyl-amino)propyl/amine

A 68,4 g (0,24 mole) d'amine obtenue en c dans 500 $cm_3$ de chlorure de méthylène et 70 $cm^3$ (0,5 mole) de triéthylamine à 0°C sont ajoutés 93 g (0,5 mole) de chlorure de tosyle par portions. Après la fin de l'addition, le mélange est laissé 6 h à température ambiante sous agitation. Le mélange réactionnel est ensuite lavé avec 600 $cm^3$ d'eau, la phase organique est séchée, évaporée à sec et le résidu est chromatographié sur colonne de silice avec le chlorure de méthylène pur puis le mélange chlorure de méthylène/méthanol/98/2. Les fractions intéressantes sont évaporées, le solide résiduel est recristallisé dans l'éthanol. Après filtration et séchage la masse obtenue est de 99,1 g (rendement 70%).

Spectre RMN : 6H $CH_3$ (doublet 0,9, 1ppm); 9H $CH_3$ tposyl (singulet 2,4 ppm); 4H $CH_2$ (triplet centré à 2,9 ppm); 2H CH (doublet 3,3 et 3,5 ppm); 12H aromatique (multiplet centré à 7,4 ppm).

e) Préparation de la N-tosyl-bis(2-tosyloxy éthyl)amine

A une solution de 185 g (0,97 mole de chlorure de tosyle dans 220 $cm^3$ de pyridine à 0°C est ajoutée une solution de 32,5 g (0,31 mole) de diéthanolamine dans 60 $cm^3$ de pyridine lentement de telle manière que la température n'excède pas 5°C. Après l'addition, le mélange est maintenu 1h à cette température puis est versé sur 220 $cm^3$ d'eau glacée sous bonne agitation. 148,4 g de précipité sont obtenus après filtration, lavage et séchage. (Rendement 85%; Rf = 0,6 silice/$CH_2Cl_2$/acétone/98/2).

Spectre RMN : 9H $CH_3$ tosyl (singulet 2,4 ppm); 4H $CH_2N$ (triplet à 3,4 ppm); 4H $CH_2O$ (triplet à 4,1 ppm); 12 H aromatiques (multiplet entre 7,1 et 7,7 ppm).

f) Préparation du N,N′, N″,N‴-tétratosyl-2,6-diméthyl-1,4,7,10-tétraazacyclododécane

65 g (0,11 mole) du composé obtenu en d dissous dans 500 $cm^3$ de DMF sec sont ajoutés goutte à goutte à 8,8g (0,22 mole) de NaH à 60% dans l'huile dans 50 $cm^3$ de DMF. L'addition se fait à température ambiante et de telle sorte que le dégagement d'hydrogène soit régulier. Après la fin de l'addition l'ensemble est chauffé à 100°C et une solution de 68,1 g (0,12 mole) de composé obtenu en e dissous dans 500 $cm^3$ de DMF sec est ajoutée goutte à goutte. Le mélange réactionnel est maintenu 24h à cette température sous bonne agitation.

Le solvant est ensuite évaporé sous vide et le résidu repris dans un mélange $CH_2Cl_2/H_2O$. La phase organique est lavée à l'eau, séchée et évaporée à sec. Le résidu (100 g) est recristallisé dans l'isopropanol puis le toluène pour donner, après filtration, lavage à l'éther isopropylique et séchage 36 g de solide blanc. (Rendement 40%; Rf = 0,5-0,6 silice/$CH_2Cl_2$/acétone/98/2).

Spectre RMN : 6H $CH_3$ (doublet à 1 et 1,2 ppm); 12H $CH_3$ tosyl (singulet 2,4 ppm); 14H $CH_2$ et CH (massif entre 3 et 4,5 ppm); 16H aromatiques (multiplet entre 7,1 et 7,7 ppm).

g) Préparation du N,N′,N″,N‴-tétratosyl-2,6-diméthyl-1,4,7,10-tétraazacyclododécane (variante)

A une suspension de 17g (28,7 mmoles) de composé obtenu en d dans 100 $cm^3$ d'éthanol à reflux est ajoutée rapidement une solution fraîchement préparée d'éthylate de sodium (60 mmoles) dans 200 $cm^3$ de DMF sec. Le mélange obtenu devient limpide et est laissé 1/2 h à reflux. Les solvants sont alors évaporés à sec, le résidu est repris avec 200 $cm^3$ de DMF et chauffé à 100 °C. A cette solution est ajoutée en 1/2h une solution de 17 g (30 mmoles) de composé obtenu en e dans 100 $cm^3$ de DMF. Le mélange réactionnel est maintenu 1 nuit à 100 °C. Le DMF est ensuite évaporé et le résidu repris dans un mélange $H_2O/CH_2Cl_2$. Le produit issu de la phase organique est chromatographié sur colonne de silice avec le mélange $CH_2Cl_2$/acétate d'éthyle/98/2 comme éluant. Le produit est recristallisé dans l'éther isopropylique et pèse après séchage 13,5 g. (Rendement 58%; Rf = 0,5-0,6 silice/$CH_2Cl_2$/acétone/98/2).

Spectre identique à celui obtenu en f.

h) Préparation du 2,6-diméthyl-1,4,7,10-tétraazacyclododécane

33 g du composé obtenu en f ou g sont mis en suspension dans 80 $cm^3$ d'acide sulfurique à 98% et chauffé 72 h à 100 °C sous atmosphère d'argon. Après refroidissement le mélange réactionnel est ajouté goutte à goutte à 1 l. d'éther éthylique à 0 °C. Le sulfate de 2,6-diméthyl-1,4,7,10-tétraazacyclododécane obtenu est filtré, repris dans l'eau, neutralisé à la soude, puis extrait avec $CH_2Cl_2$. Les phases organiques sont évaporées à sec, les 6g de solide résultant sont utilisés sans autres purifications (Rendement 74%; Rf = 0,65 alumine/butanol/eau/acide acétique/50/25/11.

Spectre RMN ($D_2O$) : 6H $CH_3$ (doublet 0,9 à 1 ppm); 14H $CH_2$ et CH (multiplet centré à 2,5 ppm).

i) Préparation de l'acide 2,6-diméthyl-1,4,7,10-tétraazacyclododécane-N,N′, N″,N‴-tétraacétique

A une solution de 3g (15 mmoles) du composé obtenu en h dans 25 $cm^3$ d'eau est ajouté un mélange de 5,7g (60 mmoles) d'acide monochloroacétique et 3,4g (60 mmoles) de potasse dans 25 $cm^3$ d'eau. Le mélange obtenu est porté à 60 °C et une solution de potasse (3,4 g, 60 mmoles) dans 25 $cm^3$ d'eau est ajoutée de telle sorte que le pH se maintienne entre 9 et 10. La durée de l'addition est de 8h. Le chauffage est maintenu 24h après la fin de l'addition de potasse. Après refroidissement le pH est ramené à 2,5 avec HCl concentré. Le précipité formé est filtré, lavé à l'eau glacée et pèse, après séchage, 3 g. (Rendement 35%; Rf = 0,33 silice/acétate d'éthyle/isopropanol/ammoniaque/12/35/30). Ce composé correspond au complexe de l'acide 2,6-diméthyl-1,4,7,10-tétraazacyclododécane-N,N′, N″, N‴-tétraacétique avec 2 KCl.

9,5 g de ce complexe sont élués avec 200 $cm^3$ d'acide acétique à 10% sur une résine échangeuse d'ions IRA 958 (OH) préalablement régénérée avec NaOH 1N et lavée à l'eau jusqu'à neutralité. Les fractions obtenues sont évaporées à sec et reprises 3 fois avec 50 $cm^3$ d'eau pour éliminer les traces d'acide acétique. Le résidu obtenu est trituré dans l'éther éthylique (100 $cm^3$) pour conduire, après filtration et séchage, à 6,3 g de solide blanc. Rendement : 89%.

Spectre RMN :($D_2O$) 6H $CH_3$ (doublet 1,4 et 1,5 ppm); 14H $CH_2$ et CH (massif complexe centré à 3,6 ppm); 8H $\underline{CH_2}COOH$ (doublet à 3,8 ppm).

Exemple 2 : Préparation du complexe de gadolinium de l'acide 2,6-diméthyl-1,4,7,10-tétraazacyclododécane-N,N′,N″,N‴-tétraacétique (sel de méthylglucamine)

Une suspension de 5,425 g (12,54 mmoles) de l'acide 2,6-diméthyl-1,4,7,10-tétraazacyclododécane-N,N′,N″,N‴-tétraacétique obtenu à l'exemple 1 i et de 2,27g de $Gd_2O_3$ (6,27 mmoles) dans 125 $cm^3$ d'eau est chauffée à 65 °C pendant 24h. Le pH est alors ajusté à 7,4 par ajout de méthylglucamine. Après dosage de $Gd^{3+}$ libre par la méthode au xylénol orange/EDTA, 650 mg d'acide 2,6-diméthyl-1,4,7,10-tétraazacyclododécane-N,N′,N″,N‴-tétraacétique (1,5 mmole) sont ajoutés pour complexer le gadolinium restant. La fin de la complexation est vérifiée par l'absence de $Gd^{3+}$ libre (dosage au xylénol orange) et de ligand libre (dosage complexométrique avec $Cu^{2+}$). Le dosage de gadolinium total dans la solution s'effectue par spectroscopie d'émission atomique en DCP sur un appareil Spectrospan 4 Beckmann. Rendement quantitatif Rf = 0,49 silice/acétate d'éthyle/isopropanol/ammoniaque/12/35/30).

Exemple 3 : Préparation de l'acide 2-hexyl-1,4,7,10-tétraazacyclododécane-N,N′,N″,N‴-tétraacétique

a) Préparation de la N-(2-hydroxyéthyl)-N-(2-hexyl-2-hydroxyéthyl)amine

50 g (0,39 mmole) d'époxy-1,2-octane sont ajoutés goutte à goutte à 250 $cm^3$ (4 moles) d'éthanolamine à 100 ° C. Le chauffage est maintenu 1h après la fin de l'addition, puis l'éthanolamine en excès est distillée sous vide. Le résidu est recristallisé dans 600 $cm^3$ d'hexane, après filtration et séchage. Le solide obtenu pèse 69g (point de fusion <45 ° C, Rendement = 93% Rf = 0,62 silice/butanol/$H_2O$/acide acétique/50/25/11).

Spectre RMN : 3H $CH_3$ (triplet 0,9 ppm); 10H $CH_2$ chaîne (singulet large à 2,2 ppm); 7H $CH_2$ et CH (2 massifs mal résolus à 2,8 et 3,8 ppm).

b) Préparation de la N-tosyl-N-(2-tosyloxy éthyl)-N-(2-hexyl-2-tosyloxyéthyl)amine

A une solution de 156 g (0,82 mole) de chlorure de tosyle dans 300 $cm^3$ de pyridine à 0 ° C sont ajoutés en 1 h par petites portions 47,3g (0,25 mole) du composé obtenu en a. Le mélange est maintenu à 0 ° C durant 2 jours, puis est versé sur un mélange glace/HCl 2/1. Le produit est extrait avec $CH_2Cl_2$, puis chromatographié sur colonne de silice avec $CH_2Cl_2$ comme éluant. La masse obtenue est de 118 g .- (Rendement 72%; Rf = 0,6 silice/$CH_2Cl_2$/acétone/98/2).

Spectre RMN : 3H $CH_3$ chaîne (triplet à 0,9 ppm); 10H $CH_2$ chaîne (singulet large à 1,3 ppm) 9H $CH_3$ tosyl (singulet à 2,4 ppm); 4H $CH_2N$ (triplet mal résolu à 3,4 ppm. 3H $CH_2O$ et CH (massif à 4,2 ppm); 12H aromatiques (massif entre 7 et 7,7 ppm).

c) Préparation de la N-tosyl-N-(2-azidoéthyl)-N-(2-hexyl-2-azidoéthyl)amine

87g (0,133 mole) de composé obtenu en b et 29,25g (0,45 mole) d'azoture de sodium sont mélangés à 350 $cm^3$ d'acétonitrile et 80 $cm^3$ d'eau. L'ensemble est porté à 65 ° C pendant 3 jours. L'acétonitrile est ensuite évaporé sous vide, le résidu est repris avec $CH_2Cl_2$; la phase organique est lavée à l'eau, séchée et évaporée; 50% d'huile jaune sont recueillis et utilisés sans autre purification (Rendement : 95%; Rf = 0,75 silice/$CH_2Cl_2$/acétone/98/2).

Spectre RMN : 3H $CH_3$ chaîne (triplet à 0,9 ppm); 10H $CH_2$ chaîne (massif à 1,4 ppm); 3H $CH_3$ tosyl (singulet à 2,4 ppm); 5H $CH_2$ et CH (massif complexe à 3,4 ppm), 4H aromatiques (massif entre 7,1 et 7,7 ppm).

Spectre I.R. $N_3$ = 2100 $cm^{-1}$ intense.

d) Préparation de la N-tosyl-N(2-aminoéthyl)-N-(2-hexyl-2-aminoéthyl)amine

71 g (0,18 mole) de diazide obtenu en c sont dissous dans 500 $cm^3$ d'éthanol auquel on ajoute 5g de charbon palladié à 50% d'humidité. La suspension est violemment agitée sous courant d'hydrogène à température ambiante pendant 24 h. Le catalyseur est éliminé par filtration; après évaporation de l'éthanol 61,5 g de diamine sont recueillie et utilisée sabs autre purification. (Rendement : quantitatif; Rf = 0,51 silice/MeOH:$NH_4OH$/95/5).

e) Préparation de la N-tosyl-N(2-tosylaminoéthyl)-N-(2-hexyl-2-tosylaminoéthyl)amine

A une solution de 61,5 g (0,18 mole) de composé obtenu en d dans 500$cm^3$ de $CH_2Cl_2$ et 52,5 $cm^3$ (0,35 mole) de triéthylamine à 0 ° C sont ajoutés par portions 68,6 g (0,36 mole) de chlorure de tosyle. Après 2 h d'agitation à température ambiante, le mélange réactionnel est traité avec 500 $cm^3$ d'eau. La phase organique est lavée à l'eau, séchée, évaporée; le résidu huileux est chromatographié sur colonne de silice avec $CH_2Cl_2$ comme éluant. L'huile obtenu après évaporation du solvent et reprise à l'éther isopropylique donne 60 g de solide blanc (point de fusion 120 ° C; rendement 51%; Rf = 0,6 silice/$CH_2Cl_2$/MeOH/98/2).

Spectre RMN : 13H chaîne hexyle (massif mal résolu centré à 1 ppm); 9H $CH_3$ tosyl(singulet à 2,4 ppm); 7H $CH_2$ et CH (massif centré à 3,1 ppm).

f) Préparation du N,N′, N″, N‴-tétratosyl-2-hexyl-1,4,7,10-tétraazacyclododécane

Un mélange de 46.5g (71,5 mmole) du composé obtenu en d ci-dessus, 41,5g (73 mmole) de composé obtenu à l'exemple 1 e et 24 g (70 mmole) de tétrabutylammonium hydrogénosulfate sont mis ensuspension dans 400 $cm^3$ de toluène et 200$cm^3$ de soude à 20%. L'ensemble est agité violemment à 70 ° C pendant 24h. Après refroidissement la phase organique est lavée à l'eau, séchée et évaporée. Le résidu est cristallisé dans l'éthanol puis chromatographié sur colonne de silice avec $CH_2$ $Cl_2$ comme éluant. 35g de

solide sont obtenus (point de fusion 154/161 ° C). Rendement 56%; Rf = 0,55 silice/$CH_2Cl_2$/acetone/98/2).

Spectre RMN : 3H $CH_3$ chaîne (triplet à 0,9 ppm); 10H $CH_2$ chaîne (massif à 1,3 ppm); 12H $CH_3$ tosyl (singulet à 2,4 ppm); 15H $CH_2$ et CH cycle (massif à 3,3 ppm); 16H aromatiques (multiplet entre 7,1 et 7,7 ppm).

g) Préparation du 2-hexyl-1,4,7,10-tétraazacyclododécane

12g (13 mmoles) du composé obtenu en f sont chauffés 24 h à 100 ° C dans 40 $cm^3$ d'acide sulfurique à 98% sous argon. Après refroidissement le mélange est versé goutte à goutte sur 500 ml d'éther éthylique à 0 ° C. Le sulfate obtenu est filtré puis neutralisé par une solution de soude à 10% et extrait avec $CH_2Cl_2$. La phase organique est séchée sur sulfate de sodium, puis évaporée à sec pour donner 2g d'un solide crème. (Rendement : 57%; Rf = 0,75 alumine/butanol/eau/acide acétique/50/25/11).

Le composé est stocké sous forme d'oxalate, en faisant réagir une solution éthanolique d'acide oxalique avec le 2-hexyl-1,4,7,10-tétraazacyclododécane pendant 1 nuit à température ambiante. L'oxalate précipite sous forme de solide blanc.

h) Préparation de l'acide 2-hexyl-1,4,7,10 -tétraazacyclododécane-N,N′,N″,N‴-tétraacétique

Une solution de 1,09 g (2,8 mmoles) d'oxalate obtenu en g dans 13 $cm^3$ d'eau et 20 ml d'éthanol est neutralise avec 470 mg (8,4 mmoles) de potasse. A cette solution est ajouté du monochloroacétate de potassium fraîchement préparé à partir de 1,063 g (11,25 mmoles) d'acide monochloroacétique, 630 mg (11,25 mmoles) de potasse dans 20 $cm^3$ d'eau.

Le mélange réactionnel est porté à 60 ° C et le pH est maintenu entre 8 et 10 par adjonction de potasse. L'addition dure 3 h au cours de laquelle 10 $cm^3$ d'eau contenant 630 mg de potasse sont ajoutés. Après 3 h de réaction 141 mg (1,5 mmole) d'acide chloroacétique et 84 mg (1,5 mmole) de potasse sont ajoutés.

Le mélange est encore maintenu 2 jours à 60 ° C. Après refroidissement et acidification à pH 2,5 (HCl 6N) la solution est passée sur colonne de résine $OH^-$ forte IRA 958. L'élution par 100 $cm^3$ d'acide formique à 10% conduit à 700 mg de produit. Les eaux de passage direct (produit non fixé) sont concentrées et retraitées sur une colonne identique. Après le même traitement 2,5 g de produit sont recueillis. (Rendement : 38,4%; Rf = 0,65 silice/éthanol/tampon/2/1).

Spectre RMN : 3H $CH_3$ chaîne (triplet 0,9 ppm); 10H $CH_2$ chaîne (massif à 1,4 ppm); 15H $CH_2$ et CH cyclique (massif à 3,3 ppm); 8H $\underline{CH_2}$ COOH (singulet à 3,9 ppm). Spectre effectué dans $D_2O$.

Exemple 4 : Préparation du complexe de gadolinium de l'acide 2-hexyl-1,4,7,10-tétraazacyclododécane-N,N′, N″,N‴-tétraacétique

488.6 mg (1 mmole) du composé obtenu à l'exemple 3 et 181,3 mg (1 meq de métal) d'oxyde de gadolinium sont mis en suspension dans 40 $cm^3$ d'eau et portés à 65 ° C pendant 2 jours. Après 2 heures la solution est limpide. Au cours de la réaction, des dosages de gadolinium libre, permettent de suivre l'évolution de la complexation. Lorsque celle-ci est totale, la solution est filtrée sur papier Millipore puis évaporé à sec et cristallisée dans l'éther éthylique. 550 mg de solide blanc sont ainsi recueillis. (Rendement : 85,5%; Rf = 0,65 ETOH/Tampon/2/1.

Exemple 5 : Préparation du complexe de gadolinium de l'acide 2-hexyl-1,4,7,10-tétraazacyclododécane-N,N′, N″,N‴-tétraacétique (sel de méthylglucamine)

488,6 mg (1 mmole) du composé obtenu à l'exemple 3 et 181,3 mg (1 meq de métal) d'oxyde de gadolinium sont mis en suspension dans 40 $cm^3$ d'eau et portés à 65 ° C pendant 12 h. A la solution limpide est ajoutée la méthylglucamine de manière à ajuster le pH à 7,4. En fonction des résultats des dosages, il est procédé à des ajouts de ligand. La fin de la complexation est vérifiée par l'absence de $Gd^{3+}$ libre (dosage au xylénol orange) et de ligand libre (dosage complexométrique avec le cuivre). Le dosage de gadolinium total dans la solution s'effectue par spectroscopie d'émission atomique sur un appareil spectrospan 4 Beckmann. Rf = 0,65 dans EtOH/Tampon/2/1.

Exemple 6 : Préparation de l'acide 2-méthyl-1,4,7,10-tétraazacyclododécane-N,N′,N″,N‴-tétraacétique

a) Préparation du N,N′-ditosyl-1,2-diaminopropane.

Dans un ballon tricol de 1 l équipé d'une agitation magnétique, d'un thermomètre et d'une garde à chlorure, 14,8 g du 1,2-diaminopropane sont solubilisés dans 500 ml de $CH_2Cl_2$ et 58 $cm^3$ de $Et_3N$.

En une heure, 80 g de chlorure de tosyle sont introduit par portions. Il est nécessaire de refroidir à l'aide d'un bain de glace pour maintenir une température de 20 ° C.

Le mélange réactionnel est ensuite agité une nuit à température ambiante.

Le mélange réactionnel est transferré dans une ampoule à décanter de 1 l puis lavé avec 2 x 250 $cm^3$ d'eau.

La fraction organique est séchée sur $Na_2SO_4$, évaporée à sec, puis cristallisée dans l'éther isopropylique.

| | |
|---|---|
| Masse obtenue | = 66 g |
| Rdt | = 86 % |
| Point de fusion | = 98/100 ° C |

| RMN | |
|---|---|
| 1 ppm doublet (3H)<br>2,4 ppm singulet (6H)<br>2.9 ppm doublet (2H) | 3,1 ppm massif (1 H)<br>5,5 ppm singulet échangeable (2H)<br>7,1 à 7,8 ppm aromatique (8H) |

CCM

$SiO_2$ éluant $CH_2Cl_2$ 90 Rf = 0,75
MeOH 10

b) Préparation du N,N′-ditosyl-bis (2-tosyloxyethyl) éthylène diamine.

Dans un ballon tricol de 500 $cm^3$ équipé d'un thermomètre, d'une garde à chlorure et d'une agitation magnétique, une solution de 162g de chlorure de tosyle dans 300 ml de pyridine est refroidie à 0 ° C, à l'aide d'un bain de glace et sel.

A cette température, en 2 h, 29,6g de bis (2-hydroxyéthyl) éthylènediamine sont ajoutés par portions. La température ne doit à aucun moment, excéder 5 ° C. Le mélange réactionnel est agité 4 h à cette température, laissé 48 h à 6/8 ° C au réfrigérateur puis 4h à température ambiante.

Le mélange réactionnel est jeté sur 1 l de glace + eau et 300 ml d'HCl concentré. Le produit est extrait avec 500 ml de $CH_2Cl_2$. Cette phase organique est séchée sur $Na_2SO_4$ puis évaporée à sec. Le résidu est repris à chaud dans 250 $cm^3$ d'éthanol. Le produit cristallise. Il est essoré sur verre fritté, séché à 60 ° C 48 h.

| | |
|---|---|
| Masse obtenue | = 107,5 g |
| Rdt | = 70% |
| Point de fusion | = 138/140 ° C |

RMN

2,4 ppm singulet (12 H)
3,3 ppm singulet + triplet (8 H)
4,2 ppm triplet (4 H)
7,2 à 7,8 ppm massif (16 H)

CCM

Plaque $SiO_2$ éluant toluène 80 Rf = 0,8
acétone 20

c) Préparation du N,N′,N″,N‴-tétratosyl-2-méthyl-1,4,7,10-tétraazacyclododécane.

Dans un ballon tricol de 1 l, une solution de 17,5 g de N,N′ ditosyldiamino 1,2-propane dans 500 ml de DMF sec, est agitée 1/4 d'heure à température ambiante. 33 g de $Cs_2CO_3$ préalablement pulvérisés sont ajoutés en suspension à cette solution. Cette suspension est portée à 55 ° C à l'aide d'un bain d'huile sous atmosphère inerte.

A cette température, et en 2 h, une solution de 35 g de N,N′-ditosyl-bis (2-tosyloxyethyl) éthylène diamine dans 200 $cm^3$ de DMF est ajoutée goutte a goutte. Après la fin de l'addition, le chauffage est poursuivi pendant 48 h. Le DMF est ensuite éliminé par distillation sous vide. Le résidu est repris dans un mélange eau/$CH_2Cl_2$.

La phase organique est séchée sur $Na_2SO_4$. Le solvant est éliminé par distillation à l'évaporateur rotatif.

Le résidu est agité à chaud, dans 200 ml d'acétate d'éthyle. Le produit attendu cristallise. Il est essoré, puis séché à 60 ° C, sous vide 24 h.

Masse obtenue = 22,5 g
Rendement = 61 %
Point de fusion = 274/275 ° C

RMN

1 ppm doublet (2 H)
2,2 ppm singulet (12 H)
3 à 3,8 ppm massif (15 H)
7,2 à 7,9 ppm massif (16 H) aromatique

CCM

$SiO_2$ éluant toluène = 80 Rf = 0,56
acétone = 20

d) Préparation du 2-méthyl-1,4,7,10-tetraazacyclododecane

Dans un tricol de 1 l équipé d'un thermomètre, d'un ballon d'argon et d'une agitation magnétique, une solution de 72,5 g du composé obtenu en c) dans 300 ml d'$H_2SO_4$ à 98% est chauffée à 100 ° C pendant 48 h à l'aide d'un bain d'huile sous atmosphère inerte.

Le mélange réactionnel est refroidi à température ambiante et additionné en 1 h à 800 ml d'$Et_2O$ refroidi à 0 ° C, à l'aide d'un bain d'éthylène glycol et de carboglace.

Le sulfate très hygroscopique précipite. Il est essoré soigneusement sous azote sur un verre fritté, puis solubilisé rapidement dans 200 ml d'eau. Cette solution est alcalinisée (NaOH en pastilles) puis extraite avec 5 x 100 ml de $CH_2Cl_2$.

Les phases organiques réunies sont séchées sur $Na_2SO_4$ puis évaporées à sec pour conduire à 15 g de produit brut très visqueux qui cristallise dans le temps.

Rendement = 90%

| RMN | | |
|---|---|---|
| $CDCl_3$<br>Spectre dans + $D_2O$ | 1,1 ppm<br>2,7 ppm | doublet (2 H)<br>2 singulets en massif (19 H dont 4 échangeables) |

CCM

Plaque $Al_2O_3$ éluant BuOH 50 Rf = 0,8
$H_2O$ 25
ACOH 11

e) Préparation du complexe de l'acide 2-méthyl-1,4,7,10-tétraazacyclododécane-N,N',N'',N'''-tétraacétique avec 2 KCl.

Dans un ballon tricol de 250 ml, une solution de 34 g d'acide chloroacétique, dans 150 $cm^3$ d'eau, est refroidie à 10 ° C, dans un bain de glace. A cette température, on ajoute de 20 g de potasse pour salifier l'acide.

Le composé obtenu en d est ensuite solubilisé dans cette solution. Le mélange réactionnel est alors porté à 65 ° C à l'aide d'un bain d'huile.

A cette température, en 6 h, en maintenant le pH entre 8 et 10, une solution de 20 g de KOH dans 50 $cm_3$ d'eau est additionnée précautionneusement.

Le chauffage est ensuite poursuivi 72 heures, puis le milieu réactionnel est acidifié à pH = 2,5 à l'aide d'HCl concentré.

Le complexe précipite. Il est essoré sur verre fritté, rincé avec 50 $cm_3$ d'eau, puis séché à 60 ° C 18 h à l'étuve.

| | |
|---|---|
| Masse obtenue | = 30 g |
| Rendement | = 66 % |
| Point de fusion | > 300 ° C |

f) Purification de l'acide 2-méthyl-1,4,7,10-tétraazacyclododécane-1,4,7,10-tétraacétique.

30 g de complexe obtenu en e sont mis en suspension en présence de 150 $cm^3$ de résine IRA 958 préalablement régénérée.

Après dissolution du complexe, cette suspension est placée en tête d'une colonne contenant 150 $cm^3$ de résine IRA 958.

L'élution est effectuée par une solution d'acide acétique à 5 % dans l'eau.

Les fractions contenant le produit attendu pur sont évaporées à sec afin d'éliminer entièrement l'acide acétique.

| | |
|---|---|
| Masse obtenue | = 18,4 g |
| Rendement | = 89 % |
| Acidité | = 100,3 % (4 équivalents) dosage avec NaOH 0,1 M. |

CCM

| | | | |
|---|---|---|---|
| $SiO_2$ | éluant ACOET | 12 | Rf = 0,36 |
| | Isopropanol | 35 | |
| | $NH_3$, $H_2O$ | 30 | |

Spectre de masse FAB pic de masse à M + 1 = 419.

Exemple 7 : Préparation d'une solution du complexe de gadolinium de l'acide 2-méthyl-1,4,7,10-tétraazacyclododécane - N,N',N'',N'''-tétraacétique (sel de méthylglucamine).

On dissout 21 g (50 mmoles) du composé obtenu à l'exemple 6 et 9,05 g (25 mmoles) d'oxyde de gadolinium dans 50 ml d'eau bidistillée et dégazée à 70 ° C. Au bout d'une heure, la dissolution est terminée, le pH est voisin de 3. Après refroidissement, on ajuste à pH 7,3 avec de la méthylglucamine. On amène à 100 ml et on filtre sur une membrane millipore 0,22 µm. On obtient ainsi une solution ayant une teneur en Gd. de 0,5 mole/l. Cette solution a une viscosité à 20 ° C inférieure à 4 mPa.s, (Gd libre non détecté).

Exemple 8 : Préparation de l'acide 2-hydroxyméthyl-1,4,7,10-tétraazacyclododécane-4,7,10-triacétique.

a) Préparation de l'acide N,N'-ditosyl-2,3-diaminopropionique.

A une solution de 46 g de soude dans 500 $cm^3$ d'eau, on ajoute 40 g de monochlorhydrate d'acide 2,3-diamino propionique sous bonne agitation. 200 $cm^3$ d'éther éthylique sont ajoutés puis, par portions, 110,5 g de chlorure de tosyle en 1 h. L'agitation est maintenue 12 h, le précipité formé est essoré puis claircé à

l'eau et à l'éther éthylique. Le solide obtenu est mis en suspension dans 1 l d'eau puis acidifié avec HCl 6N. Après filtration et lavages à l'eau et à l'éther éthylique, le solide est séché 24 h à 60 °C sous vide.
Masse obtenue : 76 g
Rendement : 65 %
Point de fusion : 200-201 °C
CCM : $SiO_2$ $CH_2Cl_2$ 80 / MeOH 20
Rf : 0,5

| RMN | | |
|---|---|---|
| - 2,4 ppm | Singulet | 6H ($CH_3$ du groupement tosyle) |
| - 2,8 ppm | Massif | 3H ($CH_2$,CH de la chaîne diamino) |
| - 3,5 à 5 ppm | Massif étalé | 3H échangeables avec $D_2O$ |
| - 7,2 à 7,8 ppm | Multiplet | 8 H aromatiques |

b) Préparation du N,N′-ditosyl-2,3-diaminopropanol.

Dans un ballon tricol de 2 l, une suspension de 40 g du composé obtenu en b) dans 600 $cm^3$ de THF est agitée à 20 °C, sous atmosphère anhydre et inerte (Argon).

Une solution de 500 ml de $BH_3$ : THF 1M est additionnée sous atmosphère inerte en 1/2 h. La température du milieu réactionnel s'élève jusqu'à 30 °C. L'agitation est poursuivie 48 h. L'hydrolyse est effectuée précautionneusement avec 20 ml d'eau. Le THF est éliminé par distillation sous vide. Le résidu est extrait dans un mélange eau/éther. La phase organique est lavée à l'eau, séchée sur $Na_2SO$, puis évaporée jusqu'à siccité. Le résidu est trituré dans l'éther isopropylique jusqu'à cristallisation. Après essorage et séchage 35 g de produit sont obtenus.
Rendement : 90 %
Point de fusion : 126-127 °C
CCM : $SiO_2$ $CH_2Cl_2$ 90 / MeOH 10
Rf : 0,6

| RMN | |
|---|---|
| - 2,7 ppm | Singulet "$CH_3$" du tosyle (6H) |
| - 3 à 3,7 ppm | Multiplet (7H dont 2 échangeables) |
| - 6,9 ppm | Triplet OH alcool échangeable (1H) |
| - 7,3 à 7,9 ppm | Multiplet aromatiques (8H) |

c) Préparation du N,N′,N″,N‴-tétratosyl-2-hydroxyméthyl-1,4,7,10-tétraazacyclododécane.

Dans un ballon tricol de 2 l, sous argon, 35 g de composé obtenu en b) sont solubilisés dans 1 l de DMF anhydre, puis additionnés de 58,6 g de $Cs_2CO_3$ anhydre.

Cette suspension est agitée 1 h à température ambiante puis portée à 65 °C à l'aide d'un bain d'huile. A cette température, une solution contenant 69 g de N, N′-ditosyl-bis (2-tosyloxyéthyl) éthylène diamine dans 600 $cm^3$ de DMF anhydre est additionnée goutte à goutte en 6 h. Le chauffage est maintenu à 65 °C une nuit; le DMF est éliminé par distillation sous vide. Le résidu est repris dans un mélange de 400 ml d'eau et 400 $cm^3$ de dichlorométhane.La phase organique est décantée, lavée avec 200 $cm^3$ d'eau, séchée sur $Na_2SO_4$, puis évaporée à sec. L'huile résiduelle est solubilisée à 80 °C dans 200 $cm^3$ de toluène puis laissée au réfrigérateur 48 h pour cristallisation. 24 g de produit sont obtenus.
Rendement : 32 %
Point de fusion : 143-145 °C
CCM : $SiO_2$ $CH_2Cl_2$ 90 / AcOEt 10
Rf : 0,5

| RMN | | |
|---|---|---|
| 2,4 ppm<br>3,2 à 4,1 ppm<br>7,2 à 8,1 ppm | Singulet<br>Massif<br>Multiplet | 12 H $CH_3$ tosyle<br>17 H $CH_2$ cycle + $CH_2$-OH<br>16 H Aromatiques |

d) Préparation du 2-hydroxyméthyl-1,4,7,10-tétraazacyclododécane.

20 g du composé obtenu en c) sont solubilisés dans 100 $cm^3$ d'$H_2SO_4$ à 98 %. Cette solution est chauffée à 100° C pendant 48 h sous atmosphère inerte. Le mélange réactionnel est refroidi puis ajouté goutte à goutte à 1 l d'éther éthylique réfrigéré à l'aide d'un bain de carboglace/acétone. Le précipité de sulfate de l'amine est essoré sur verre fritté puis claircé à l'éther éthylique. Le solide est immédiatement solubilisé dans 200 $cm^3$ d'eau, la solution est alcalinisée avec NaOH à pH > 12, et évaporée à sec. Après séchage sous vide du solide résiduel en présence de $P_2O_5$, le produit est extrait de 2 x 100 $cm^3$ de THF à reflux. Les fractions d'extraction évaporées conduisent à une huile incolore.
Masse obtenue : 4,5 g de base
Rendement : 90%
CCM : $Al_2O_3$ BuOH 50 / Eau 25 / AcOH 11
Rf : 0,8

| RMN (Spectre $CDCl_3$) | |
|---|---|
| 2,8 ppm<br>3,8 ppm | Singulet (17 H) + triplet<br>Singulet échangeable (5 H) |

e) Préparation de l'acide 2-hydroxyméthyl-1,4,7,10-tétraazacyclododécane -4,7,10-triacétique.

Dans un tricol de 250 $cm^3$ équipé d'une agitation magnétique chauffante, d'une sonde de température et d'une électrode de pH-mètre reliée à un pH-mètre analogique et d'un système d'addition de réactif corrélé au pH du milieu, une solution de 8,5 g du composé obtenu en d), 15,8g d'acide 2-chloroacétique et 100 ml d'eau est neutralisée jusqu'à pH = 9,5 à l'aide d'une solution de 15,8 g de potasse dans 50 $cm^3$ d'eau. Le mélange réactionnel est alors porté à 50° C à l'aide d'un bain d'huile durant 72 h. Le pH étant par ailleurs ajusté en permanence à 9,5 à l'aide d'une solution de potasse. Le mélange est refroidi, acidifié jusqu'à pH = 5, dilué à 500 $cm^3$ et élué sur une colonne de 500 $cm^3$ de résine IRA 958 échangeuse d'anions forte préalablement régénérée. Les produits d'alkylation se fixent sur la résine. Celle-ci est rincée à l'eau puis éluée avec des fractions d'acide acétique à 5%. Les fractions sont évaporées à sec. Le résidu est une poudre brute qui est purifiée sur colonne HPLC préparative de diamètre 40 chargée avec une silice greffée RP.18.
Masse obtenue : 3,5 g de produit pur
Rendement : 22 %
Point de fusion : 142-144° C
CCM : $SiO_2$ AcOEt 12 / Isopropanol 35 / $NH_4OH$ 30
Rf : 0,35
Acidité : 198,7 % (2 vagues)
Dosage avec NaOH 0,1 M - corrigé de $H_2O$
Spectre de masse FAB pic à M + 1 = 377

<u>Exemple 9 : Préparation d'une solution du complexe de gadolinium de l'acide 2-hydroxyméthyl-1,4,7,10-tétraazacyclododécane-4,7,10 triacétique.</u>

Une suspension de 11,05 g de l'acide 2-hydroxyméthyl-1,4,7,10-tétraazacyclododécane-4,7,10-triacétique et de 5,07 g d'oxyde de gadolinium dans l'eau bidistillée est chauffée à 80° C pendant 1 h.

Après refroidissement, le pH est ajusté à 7,3 par ajout de soude et le volume ajusté à 100 ml. Le dosage du gadolinium total est réalisé par spectiométrie d'émission atomique (0,28 M $l^{-1}$).

Exemple 10 : Préparation de l'acide 2-hydroxyméthyl-1,4,7,10-tétraazacyclododécane - 1,4,7,10-tétra acétique.

Dans un réacteur de 50 $cm^3$ équipé d'une agitation magnétique, une solution de 0,7g de l'acide 2-hydroxyméthyl-1,4,7,10-tétraazacyclododécane-4,7,10-triacétique et 0,28 g d'acide chloracétique dans 15 $cm^3$ d'eau est chauffée à 70 ° C.

Le pH amené à 10,5 à l'aide d'une solution de potasse, est maintenu à cette valeur 48 heures à 70 ° C.

En fin de réaction, le pH est amené à 5, puis la solution est éluée sur une résine IRA 958.

Le ligand est chromotographié sur résine par élution à l'acide acétique à 5%.

Après évaporation à sec, le produit est purifié par HPLC préparative (silice greffée RP18).

0,15 g de ligand sont ainsi récupérés avec un rendement de 18%.

| | | | |
|---|---|---|---|
| CCM (silice) éluant | Acétate d'éthyle | 12 | |
| | Isopropanol | 35 | Rf = 0,25 |
| | $NH_3.H_2O$ | 30 | |

Spectre de masse FAB pic à M + 1 = 435.

Exemple 11 : Préparation de l'acide 2-(2-hydroxyéthyl) -1,4,7,10-tétraazacyclododécane-N,N′,N″,N‴ tétraacétique.

Ce ligand est obtenu selon le mode opératoire décrit aux exemples 8 et 10, pour la synthèse de l'acide 2-hydroxyméthyl 1,4,7,10-tétraazacyclododécane -N,N′,N″,N‴-tétraacétique, à partir de l'acide 3,4-diamino butanoique (S Kasina et al., 3 Med Chem, 29, 1933, 1986).

Exemple 12 : Préparation de l'acide 2-méthyl-1,4,7,10,13-pentaazacyclopentadécane-4,7,10,13-tétraacétique (produit 12a) et de l'acide 2-méthyl-1,4,7,10,13-pentaazacyclopentadécane-1,4,7,10,13-pentaacétique (produit 12b).

a) Préparation du 1,4,7,10,13-pentatosyl-2-méthyl-1,4,7,10,13-pentaazacyclopentadécane.

Dans un tricol de 2 l muni d'un réfrigérant, d'une agitation mécanique, on charge 35,7 g (0,093 mole) de N,N′-ditosyl-1,2-diaminopropane, 75,7 g de carbonate de césium (0,23 mole) et 800 ml de DMF.

Le milieu est mis sous argon puis chauffé à 75 ° C.

Une solution de 83g (0,012 mole) de 1,11-mésyloxy-3,6,9-tritosyl-3,6,9-triazaundécane synthétisé selon Richman and Atkins Organic Synthesis 58, p 86, dans 700 ml de DMF est ajoutée en 4 heures à 75 ° C.

Le milieu réactionnel est maintenu 48 heures à 75 ° C, puis est filtré et la solution est concentrée à sec.

Le résidu est repris dans 700 ml d'éthanol, le solide est filtré puis repris à chaud dans 800 ml de toluène.

Le solide est filtré à température ambiante puis séché à 60 ° C.

On obtient : M : 45,4 g
Rd : 49%

Analyse : CCM : $SiO_2$ 60 F254 Merck
Eluant $CH_2Cl_2$ / Acétone 98.2 Rf 0,45
Spectre de masse
Méthode DCI ($NH_3$)
Pic de masse à 999.

b) Préparation du 2-méthyl-1,4,7,10,13-pentaazacyclopentadécane.

44 g (0,044 mole) de composé obtenu en a) sont maintenus, 72 heures à 100 ° C dans 130 ml d'acide sulfurique concentré.

Après refroidissement, le milieu réactionnel est versé dans un mélange à 0 ° C de 250 ml d'éther éthylique et 250 ml d'éthanol.

Le solide est filtré puis solubilisé dans 250 ml d'eau et traité au noir de carbone.

La solution est alcalinisée par de la soude puis extraite par $CH_2Cl_2$.

La solution organique est séchée sur $Na_2SO_4$ et évaporée à sec.

L'amine ainsi obtenue peut être utilisée telle quelle ou bien sous forme de chlorhydrate.

On obtient : M = 8,4 g sous forme base
M = 13,2 g sous forme 5 HCl  Rd : 72,8%

Analyse du chlorydrate

CCM : $Al_2O_3$ F 254 Merck
Eluant : Ethanol/Isopropylamine 80.20
révélateur Iode  Rf : 0,85
RMN : = 1,7 ppm 3 H $CH_3$
= 3,7 ppm 19 H $CH_2$ et CH.

c) Préparation de l'acide 2-méthyl-1,4,7,10,13-pentaazacyclopentadécane-4,7,10,13-tétraacétique (produit 12a) et de l'acide 2-méthyl-1,4,7,10,13-pentaazacyclopentadécane-1,4,7,10,13-pentaacétique (produit 12b).

Dans un tricol de 500 ml, une solution de 25,7g (0,27 mole) d'acide chloracétique dans 50 ml d'eau est neutralisée à pH = 5 et à T < 10 ° C par de la potasse 5 M.

A cette solution, 10g de composé obtenu en b) (10,043 m mole) en solution dans 20 ml d'eau sont additionnés.

Le milieu est porté à 55 ° C et 50 ml de potasse 5 M sont ajoutés à pH 8,5 - 9,5 en 48 heures.

En fin d'addition, le chauffage est maintenu une nuit.

Le mélange réactionnel est refroidi et acidifié à pH = 3.

La solution est ensuite éluée sur 200 ml de résine DOWEX 50 w.

Par élution de la résine par 1 l d'une solution d'amonniaque 1 M, on recueille 20 g de produit brut.

Le produit brut solubilisé dans 150 ml d'eau est élué sur 250 ml de résine IRA 958.

La résine est lavée à l'eau puis éluée avec 2 l d'acide acétique 0,1 M puis éluée avec 2 l d'acide acétique 0,8 M.

Par concentration de l'acide acétique 0,1 M, on obtient 9 g de produit 12a brut.

Par concentration de l'acide acétique 0,8 M, on obtient 2,5 g de produit 12b brut.

Les produits 12a et 12b sont ensuite purifiés par HPLC préparative sur silice RP18.

On obtient : Produit 12a : M = 5 g
Produit 12b : M = 1,1 g
Rendement : 30%

Analyse : CCM : $SiO_2$ 60 F 254 Merck
Eluant : Acétate d'éthyle/Isopropanol/$NH_3$ (30%) / 12 - 35 - 30 /
Révélateur : Iode
Produit 12a Rf : 0,4
Produit 12b rf : 0,27
Dosage d'eau :
Produit 12a : KF : 1,8%
Produit 12b : KF : 2,8%
Dosage d'acidité par NaOH 0,1 M
Produit 12a : 2 acidités dosées
Titre : 99,6%
Produit 12b : 3 acidités dosées
Titre : 97,3%
Spectres de masse FAB
12a pic à M + 1 = 462
12b pic à M + 1 = 520

**Revendications**

**Revendications pour les Etats contractants suivants : AT, BE, CH, DE, FR, GB, GR, IT, LI, LU, NL, SE**

**1.** Ligands de formule :

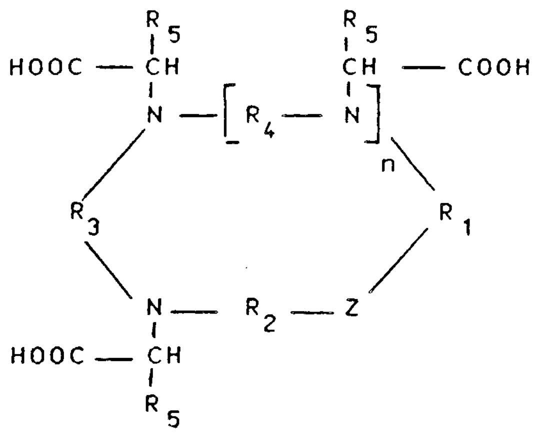

I

dans laquelle
$R_1$ représente un radical de formule

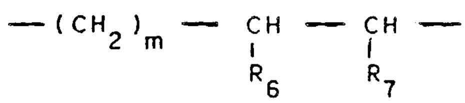

$R_6$ étant choisi parmi un groupe alkyle en $C_1$-$C_{14}$, un groupe hydroxyalkyle en $C_1$-$C_4$ et un groupe polyhydroxyalkyle en $C_1$-$C_4$ et un groupe de formule

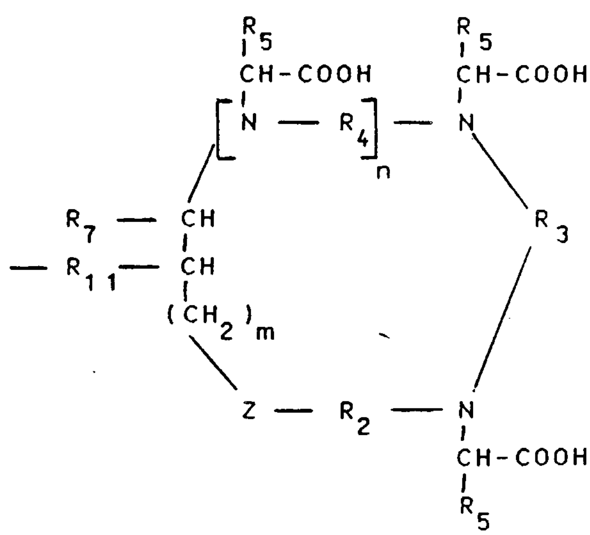

$R_{11}$ étant choisi parmi les groupes A et les groupes de formule $-(CH_2)_t$ - Y - A - Y-$(CH_2)_t$-
A étant choisi parmi les groupes alkylène en $C_1$-$C_8$, hydroxyalkylène en $C_1$-$C_8$ et polyhydroxyalkylène en $C_1$ - $C_8$,
Y étant choisi parmi

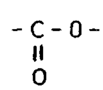

et -O- et t = 1 à 4,
$R_7$ étant choisi parmi un atome d'hydrogène, un groupe alkyle en $C_1$-$C_{14}$, un groupe hydroxyalkyle en

$C_1$-$C_4$ et un groupe polyhydroxyalkyle en $C_1$-$C_4$,
m = 0 ou 1
$R_2$,$R_3$,$R_4$ identiques ou différents représentent un radical de formule

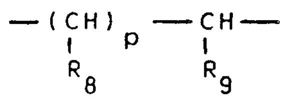

$R_8$ et $R_9$ identiques ou différents étant choisis parmi un atome d'hydrogène, un groupe alkyle en $C_1$-$C_{14}$, un groupe hydroxyalkyle en $C_1$-$C_4$, et un groupe polyhydroxyalkyle en $C_1$-$C_4$,
p = 1 ou 2
n = 0,1 ou 2 et
$R_5$ est choisi parmi un atome d'hydrogène, un groupe alkyle en $C_1$-$C_4$, un groupe hydroxyalkyle en $C_1$-$C_4$ et un groupe polyhydroxyalkyle en $C_1$-$C_4$, et
Z est choisi parmi un atome d'oxygène et un groupe de formule

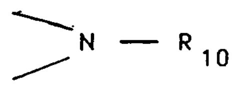

$R_{10}$ étant choisi parmi un atome d'hydrogène, un groupe alkyle en $C_1$-$C_{14}$, un groupe hydroxyalkyle en $C_1$-$C_4$, un groupe polyhydroxyalkyle en $C_1$-$C_4$, un goupe de formule

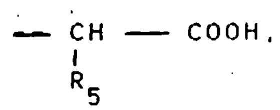

$R_5$ ayant la signification donnée précédemment, et un groupe de formule

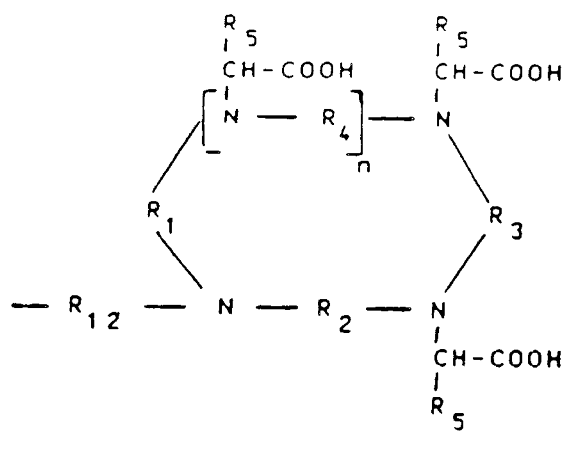

$R_{12}$ étant choisi parmi les groupes alkylène on $C_1$-$C_8$, hydroxyalkylène en $C_1$-$C_8$ et polyhydroxyalkylène en $C_1$ - $C_8$,
ainsi que leurs sels.

2. Ligands de formule I selon la revendication 1, dans laquelle m est égal à 0.

3. Composé selon la revendication 1 qui est l'acide 2,6-diméthyl-1,4,7,10-tétraazacyclododécane-N,N',N'',N'''-tétraacétique.

4. Composé selon la revendication 1 qui est l'acide 2-hexyl-1,4,7,10-tétraazacyclododécane-N,N',N'',N'''-tétraacétique.

**5.** Composé selon la revendication 1 qui est l'acide 2-méthyl-1,4,7,10-tétraazacyclododécane-N,N',N",N"'-tétraacétique.

**6.** Composé selon la revendication 1 qui est l'acide 2-hydroxyméthyl-1, 4,7,10-tétraazacyclododécane-4,7,10-triacétique.

**7.** Composé selon la revendication 1 qui est l'acide 2-hydroxyméthyl-1,4,7,10-tétraazacyclododécane-1,4,7,10-tétra acétique.

**8.** Composé selon la revendication 1 qui est l'acide 2-(2-hydroxyéthyl)-1,4,7,10-tétraazacyclododécane-1,4,7,10-tétra acétique.

**9.** Procédé de préparation d'un composé selon la revendication 1 ou 2, caractérisé en ce que l'on fait réagir un composé de formule

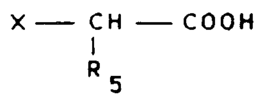

II

dans laquelle $R_5$ à la signification donnée à la revendication 1 et X représente un groupe labile, ou un aldéhyde de formule

$R_5-CHO$ IIa

dans laquelle $R_5$ a la signification donnée à la revendication 1 en présence d'acide cyanhydrique ou d'ions cyanure, avec une amine cyclique de formule

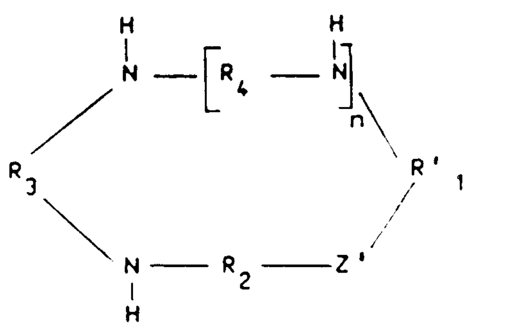

III

dans laquelle $R_2$, $R_3$, $R_4$, et n ont la signification donnée ci-dessus,
$R'_1$ représente un radical de formule

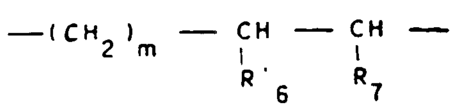

$R'_6$ étant choisi parmi un groupe alkyle en $C_1$-$C_{14}$ , un groupe hydroxyalkyle en $C_1$-$C_4$, un groupe polyhydroxyalkyle en $C_1$-$C_4$ et un groupe de formule

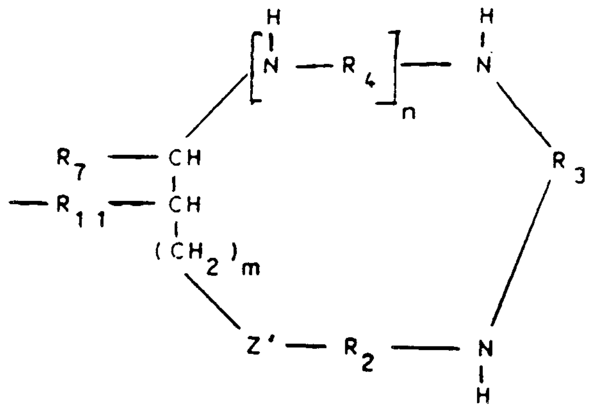

$R_2$, $R_3$, $R_4$, $R_7$, $R_{11}$, m, n, ayant la signification donnée à la revendication 1, et Z' est choisi parmi un atome d'oxygène et un groupe de formule

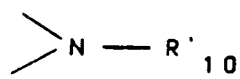

$R'_{10}$ étant choisi parmi un atome d'hydrogène, un groupe alkyle en $C_1$-$C_{14}$, un groupe hydroxyalkyle en $C_1$-$C_4$, un groupe polyhydroxyalkyle en $C_1$-$C_4$, et un groupe de formule

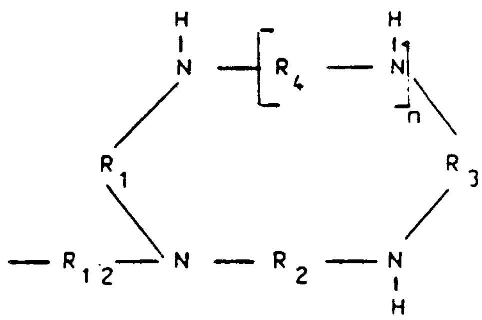

$R_1$, $R_2$, $R_3$, $R_4$, $R_{12}$ et n ayant la signification donnée ci-dessus.

**10.** Procédé selon la revendication 9, caractérisé en ce que l'on prépare le composé de formule III par réaction d'une polyamine de formule

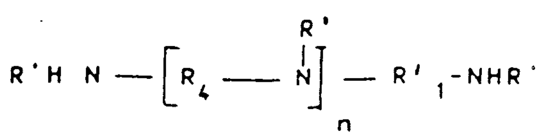
IV

dans laquelle $R_1$ et $R_2$ ont la signification donnée à la revendication 9 et R' représente un groupe tosyle, mésyle ou benzène sulfonyle
avec un composé de formule

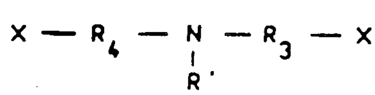
V

dans laquelle $R_3$, $R_4$ et R' ont la signification donnée à la revendication 9 et X représente un groupe labile.

**11.** Procédé selon la revendication 10 caractérisé en ce que l'on prépare le composé de formule III par réaction d'une diamine de formule

$R'HN-R'_1-NH-R'$ X

dans laquelle $R'_1$ et R' ont la signification donnée à la revendication 10, avec un composé de formule

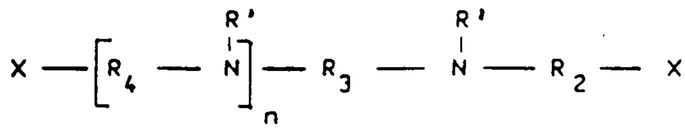 XI

dans laquelle n, $R_2$, $R_3$, $R_4$ et R' ont la signification donnée à la revendication 10 et X représente un groupe labile.

**12.** Complexes formés par des ligands de formule I selon l'une quelconque des revendications 1 à 3, avec des ions métalliques choisis parmi les ions des lanthanides (numéros atomiques 57 à 71), les ions des métaux de transition (numéros atomiques 21 à 29) et les ions des métaux de numéro atomique 55, 56, 82 et 83, ainsi que les sels de ces complexes avec des bases minérales ou organiques pharmaceutiquement acceptables ou des aminoacides basiques.

**13.** Complexes selon la revendication 12, dans lesquels l'ion métallique est choisi parmi le gadolinium, l'europium, le dysprosium, le fer ($Fe^{3+}$) et le manganèse ($Mn^{2+}$).

**14.** Complexes selon la revendication 13 qui est le sel de méthylglucamine du complexe de gadolinium de l'acide 2,6-dimèthyl-1,4,7,10-tétraazacyclododécane-N,N',N",N"'-tétraacétique.

**15.** Complexe selon la revendication 13 qui est le sel de méthylglucamine du complexe de gadolinium de l'acide 2-hexyl-1,4,7,10-tétraazacyclododécane-N,N',N",N"'-tétraacétique.

**16.** Complexe selon la revendication 13 qui est le sel de méthylglucamine du complexe de gadolinium de l'acide 2-méthyl-1,4,7,10-tétraazacyclododécane - N,N',N",N"'-tétraacétyque.

**17.** Complexe selon la revendication 13 qui est le complexe de gadolinium de l'acide 2-hydroxyméthyl-1,4,7,10-tétraazacyclododécane-4,7,10 triacétique.

**18.** Composition de diagnostic administrable à l'homme, caractérisée en ce qu'elle comprend au moins un complexe selon l'une quelconque des revendications 12 à 17.

**19.** Composition selon la revendication 18, constituée par une solution dans un solvant aqueux du complexe.

**Revendications pour l'Etat contractant suivant : ES**

**1.** Procédé de préparation de ligands de formule :

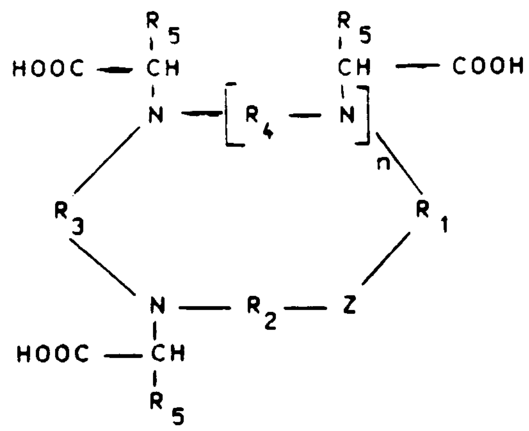

I

dans laquelle
$R_1$ représente un radical de formule

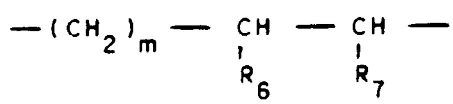

$R_6$ étant choisi parmi un groupe alkyle en $C_1$-$C_{14}$, un groupe hydroxyalkyle en $C_1$-$C_4$ et un groupe polyhydroxyalkyle en $C_1$-$C_4$ et un groupe de formule

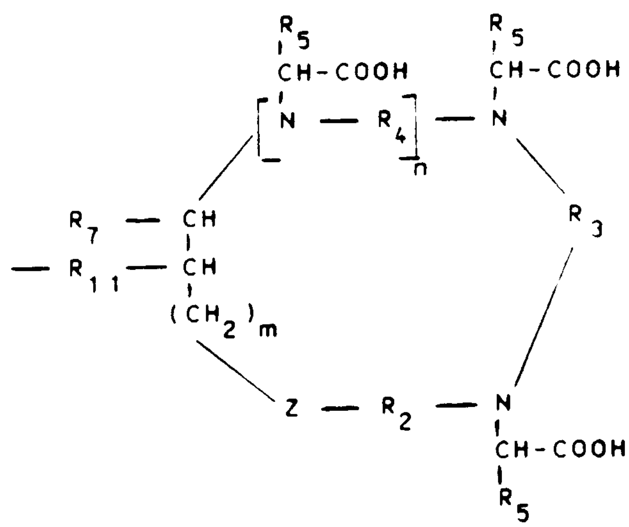

$R_{11}$ étant choisi parmi les groupes A et les groupes de formule $-(CH_2)_t$ - Y - A - Y-$(CH_2)_t$-
A étant choisi parmi les groupes alkylène en $C_1$-$C_8$, hydroxyalkylène en $C_1$-$C_8$ et polyhydroxyalkylène en $C_1$ - $C_8$,
Y étant choisi parmi

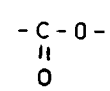

et -O- et t = 1 à 4,
$R_7$ étant choisi parmi un atome d'hydrogène, un groupe alkyle en $C_1$-$C_{14}$, un groupe hydroxyalkyle en $C_1$-$C_4$ et un groupe polyhydroxyalkyle en $C_1$-$C_4$,
m = 0 ou 1

$R_2$,$R_3$,$R_4$ identiques ou différents représentent un radical de formule

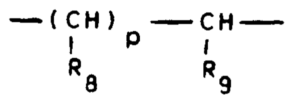

$R_8$ et $R_9$ identiques ou différents étant choisis parmi un atome d'hydrogène, un groupe alkyle en $C_1$-$C_{14}$, un groupe hydroxyalkyle en $C_1$-$C_4$, et un groupe polyhydroxyalkyle en $C_1$-$C_4$,
p = 1 ou 2
n = 0,1 ou 2 et
$R_5$ est choisi parmi un atome d'hydrogène, un groupe alkyle en $C_1$-$C_4$, un groupe hydroxyalkyle en $C_1$-$C_4$ et un groupe polyhydroxyalkyle en $C_1$-$C_4$, et
Z est choisi parmi un atome d'oxygène et un groupe de formule

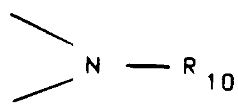

$R_{10}$ étant choisi parmi un atome d'hydrogène, un groupe alkyle en $C_1$-$C_{14}$, un groupe hydroxyalkyle en $C_1$-$C_4$, un groupe polyhydroxyalkyle en $C_1$-$C_4$, un goupe de formule

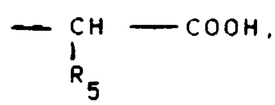

$R_5$ ayant la signification donnée précédemment, et un groupe de formule

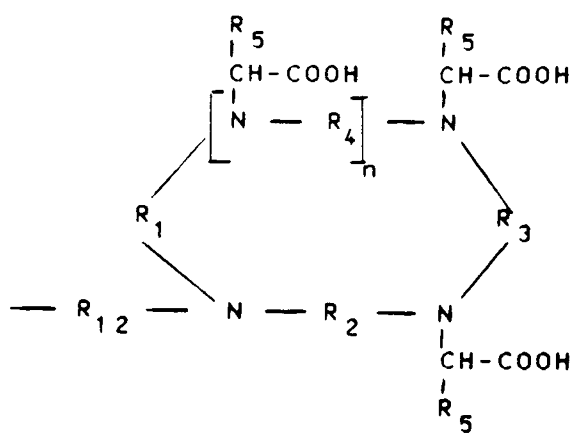

$R_{12}$ étant choisi parmi les groupes alkylène en $C_1$-$C_8$, hydroxyalkylène en $C_1$-$C_8$ et polyhydroxyalkylène en $C_1$ - $C_8$,
ainsi que leurs sels, caractérisé en ce que l'on fait réagir un composé de formule

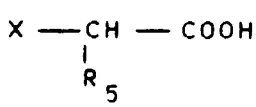

II

dans laquelle $R_5$ à la signification donnée ci-dessus et X représente un groupe labile, ou un aldéhyde de formule

$R_5$—CHO IIa

dans laquelle $R_5$ a la signification donnée ci-dessus en présence d'acide cyanhydrique ou d'ions cyanure, avec une amine cyclique de formule

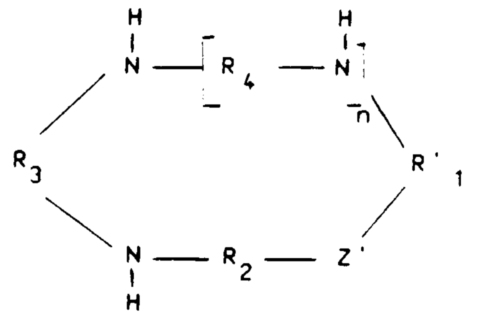

III

dans laquelle $R_2$, $R_3$, $R_4$, et n ont la signification donnée ci-dessus,
$R'_1$ représente un radical de formule

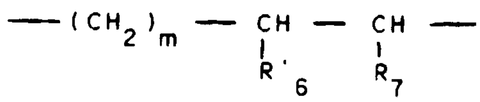

$R'_6$ étant choisi parmi un groupe alkyle en $C_1$-C$1_4$ , un groupe hydroxyalkyle en $C_1$-$C_4$, un groupe polyhydroxyalkyle en $C_1$-$C_4$ et un groupe de formule

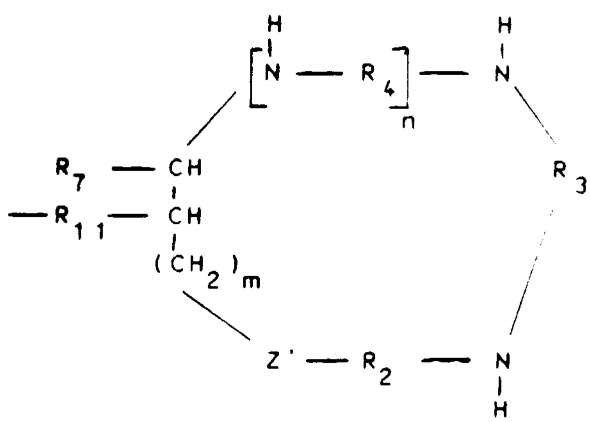

$R_2$, $R_3$, $R_4$, $R_7$, $R_{11}$, m, n, ayant la signification donnée ci-dessus, et Z′ est choisi parmi un atome d'oxygène et un groupe de formule

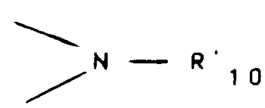

$R'_{10}$ étant choisi parmi un atome d'hydrogène, un groupe alkyle en $C_1$-$C_{14}$, un groupe hydroxyalkyle en $C_1$-$C_4$, un groupe polyhydroxyalkyle en $C_1$-$C_4$, et un groupe de formule

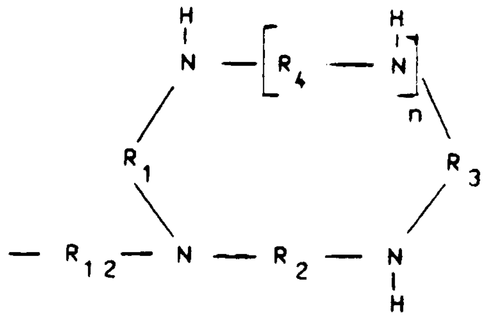

$R_1$, $R_2$, $R_3$, $R_4$, $R_{12}$ et n ayant la signification donnée ci-dessus.

**2.** Procédé selon la revendication 1, caractérisé en ce que l'on prépare le composé de formule III par réaction d'une polyamine de formule

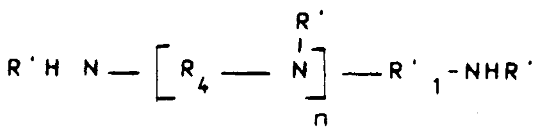

IV

dans laquelle $R_1$ et $R_2$ ont la signification donnée à la revendication 1 et R′ représente un groupe tosyle, mésyle ou benzène sulfonyle
avec un composé de formule

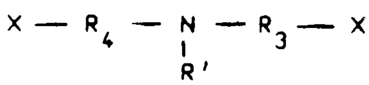

V

dans laquelle $R_3$, $R_4$ et R′ ont la signification donnée à la revendication 1 et X représente un groupe labile.

**3.** Procédé selon la revendication 1, caractérisé en ce que l'on prépare le composé de formule III par réaction d'une diamine de formule

$R'HN—R'_1—NH—R'$ X

dans laquelle $R'_1$ et R′ ont la signification donnée à la revendication 1, avec un composé de formule

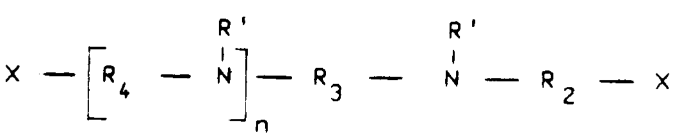

XI

dans laquelle n, $R_2$, $R_3$, $R_4$ et R′ ont la signification donnée à la revendication 1 et X représente un groupe labile.

**4.** Procédé de préparation de complexes formés par des ligands de formule I définie à la revendication 1, avec des ions métalliques choisis parmi les ions des lanthanides (numéros atomiques 57 à 71), les ions des métaux de transition (numéros atomiques 21 à 29) et les ions des métaux de numéro atomique 55, 56, 82 et 83, ainsi que les sels de ces complexes avec des bases minérales ou organiques pharmaceutiquement acceptables ou des aminoacides basiques, caractérisé en ce que l'on fait réagir le ligand avec un sel ou un oxyde des métaux dans un solvent aqueux et éventuellement on neutralise pour former un sel.

**5.** Procédé de préparation d'une composition de diagnostic administrable à l'homme, caractérisé en ce que l'on met un complexe tel que défini à la revendication 4 sous une forme pharmaceutiquement acceptable.

## Claims

**Claims for the following Contracting States : AT, BE, CH, DE, FR, GB, GR, IT, LI, LU, NL, SE**

**1.** Ligands of formula:

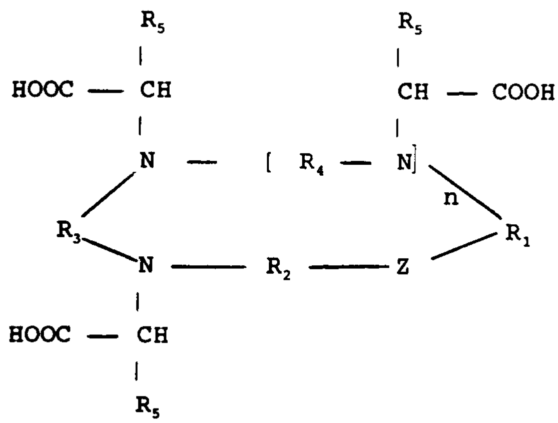

I

in which
$R_1$ represents a radical of formula

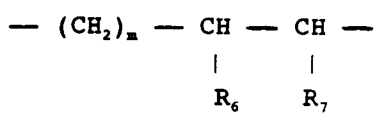

$R_6$ being selected from a $C_1$-$C_{14}$ alkyl group, a $C_1$-$C_4$ hydroxyalkyl group and a $C_1$-$C_4$ polyhydroxyalkyl group and a group having the formula

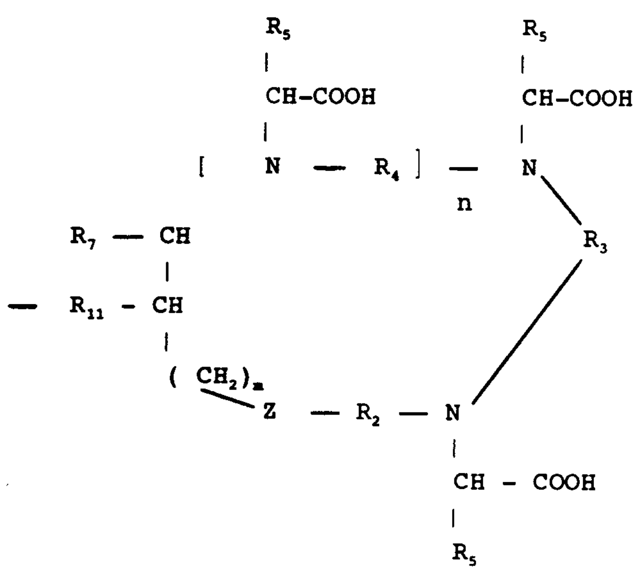

$R_{11}$ being selected from groups A and groups of formula $-(CH_2)_t$ - Y - A - Y - $(CH_2)_t$-

A being selected from $C_1$-$C_8$ alkylene groups, $C_1$-$C_8$ hydroxalkylene and $C_1$-$C_8$ polyhydroxyalkylene groups,
Y being selected from

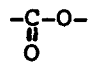

and -O- and t = 1 to 4,
$R_7$ being selected from a hydrogen atom, a $C_1$-$C_{14}$ alkyl group, a $C_1$-$C_4$ hydroxyalkyl group and a $C_1$-$C_4$ polyhydroxyalkyl group,
m = 0 or 1
$R_2$,$R_3$,$R_4$, which may be identical or different, representing a radical of formula

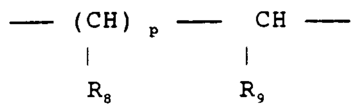

$R_8$ and $R_9$, which may be identical or different, being selected from a hydrogen atom, a $C_1$-$C_{14}$ alkyl group, a $C_1$-$C_4$ hydroxyalkyl group and a $C_1$-$C_4$ polyhydroxyalkyl group,
p = 1 or 2
n = 0,1 or 2 and
$R_5$ is selected from a hydrogen atom, a $C_1$-$C_4$ alkyl group, a $C_1$-$C_4$ hydroxyalkyl group and a $C_1$-$C_4$ polyhydroxyalkyl group, and
Z is selected from a hydrogen atom and a group having the formula

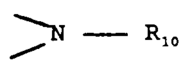

$R_{10}$ being selected from a hydrogen atom, a $C_1$-$C_{14}$ alkyl group, a $C_1$-$C_4$ hydroxyalkyl group, a $C_1$-$C_4$ polyhydroxyalkyl group, a group of formula

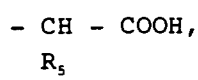

$R_5$ having the meaning above, and a group having the formula

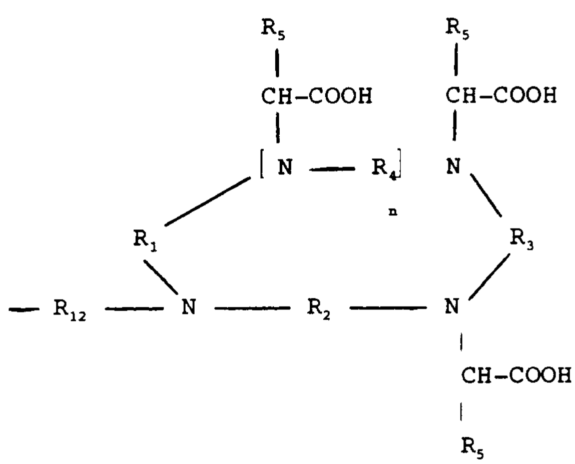

$R_{12}$ being selected from $C_1$-$C_8$ alkylene, $C_1$-$C_8$ hydroxyalkylene and $C_1$-$C_8$ polyhydroxyalkylene groups,
and their salts.

**2.** Ligands of formula I according to claim 1, in which m is equal to 0.

**3.** A compound according to claim 1, which is 2,6-dimethyl-1,4,7,10-tetraazacyclododecane-N,N',N",N"'-tetraacetic acid.

**4.** A compound according to claim 1, which is 2-hexyl-1,4,7,10-tetraazacyclododecane-N,N',N",N"'-tetraacetic acid.

**5.** A compound according to claim 1, which is 2-methyl-1,4,7,10-tetraazacyclododecane-N,N',N",N"'-tetraacetic acid.

**6.** A compound according to claim 1, which is 2-hydroxymethyl-1,4,7,10-tetraazacyclododecane-4,7,10-triacetic acid.

**7.** A compound according to claim 1, which is 2-hydroxymethyl-1,4,7,10-tetraazacyclododecane-1,4,7,10-tetraacetic acid.

**8.** A compound according to claim 1, which is 2-(2-hydroxyethyl)-1,4,7,10-tetraazacyclododecane-1,4,7,10-tetraacetic acid.

**9.** A method for preparing a compound according to claim 1 or 2, characterised in that a compound of formula

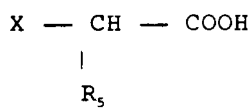

II

in which $R_5$ has the meaning indicated in claim 1 and X represents a labile group or an aldehyde of formula

$R_5 - CHO$ IIa

in which $R_5$ has the meaning given in claim 1 is reacted in the presence of hydrocyanic acid or cyanide ions, with a cyclic amine of formula

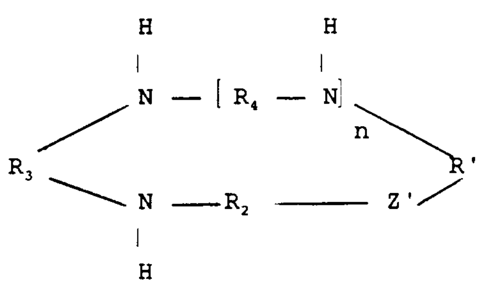

III

in which $R_2$, $R_3$, $R_4$ and n have the meanings given above,
$R'_1$ represents a radical of formula

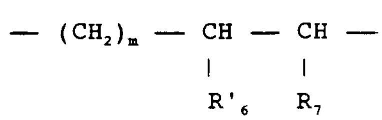

$R'_6$ being selected from a $C_1$-$C_{14}$ alkyl group, a $C_1$-$C_4$ hydroxyalkyl group, a $C_1$-$C_4$ polyhydroxyalkyl group and a group having the formula

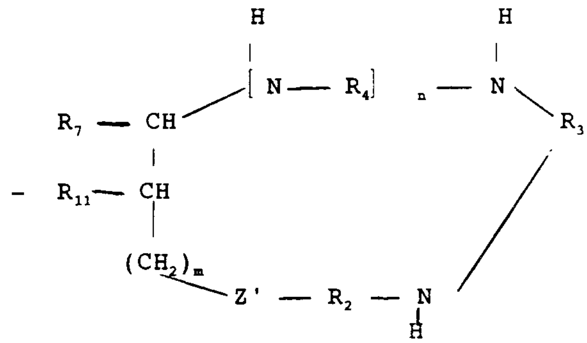

$R_2$, $R_3$, $R_4$, $R_7$, $R_{11}$, m, n having the meanings given in claim 1 and Z' being selected from an oxygen atom and a group having the formula

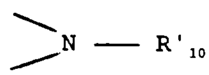

$R'_{10}$ being selected from a hydrogen atom, a $C_1$-$C_{14}$ alkyl group, a $C_1$-$C_4$ hydroxyalkyl group, a $C_1$-$C_4$ polyhydroxyalkyl group and a group having the formula

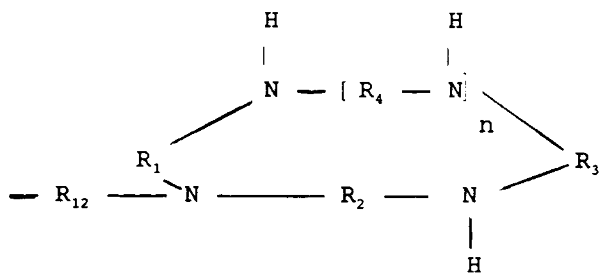

$R_1$, $R_2$, $R_3$, $R_4$, $R_{12}$ and n having the meanings given above.

**10.** A method according to claim 9, characterised in that the compound of formula III is prepared by reacting a polyamine of formula

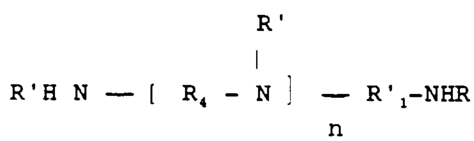 IV

in which $R_1$ and $R_2$ have the meanings given in claim 9 and R' represents a tosyl, mesyl or benzene sulphonyl group
with a compound of formula

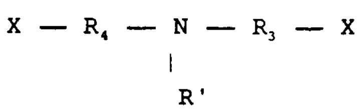 V

in which $R_3$, $R_4$ and R' have the meanings given in claim 9 and X represents a labile group.

**11.** A method according to claim 10, characterised in that the compound of formula III is prepared by reacting a diamine of formula

$R'HN - R'_1 - NH - R'$ X

in which $R'_1$ and R' have the meanings given in claim 10, with a compound of formula

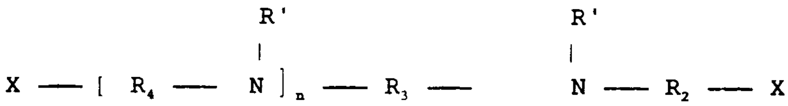

XI

in which n, $R_2$, $R_3$, $R_4$ and R' have the meanings given in claim 10 and X represents a labile group.

**12.** Complexes formed by ligands of formula I according to any one of claims 1 to 8 with metal ions selected from ions of the lanthanides (atomic numbers 57 to 71), ions of the transition metals (atomic numbers 21 to 29) and the ions of metals of atomic numbers 55, 56, 82, 83, and the salts of these complexes with pharmaceutically acceptable mineral or organic bases or basic amino acids.

**13.** Complexes according to claim 12, in which the metal ion is selected from gadolinium, europium, dysprosium, iron ($Fe^{3+}$) and manganese ($Mn^{2+}$).

**14.** A complex according to claim 13 which is the methylglucamine salt of the gadolinium complex of 2,6-dimethyl-1,4,7,10-tetraazacyclododecane-N,N',N",N"'-tetraacetic acid.

**15.** A complex according to claim 13 which is the methylglucamine salt of the gadolinium complex of 2-hexyl-1,4,7,10-tetraazacyclododecane-N,N',N",N"'-tetraacetic acid.

**16.** A complex according to claim 13 which is the methylglucamine salt of the gadolinium complex of 2-methyl-1,4,7,10-tetraazacyclododecane-N,N',N",N"'-tetraacetic acid.

**17.** A complex according to claim 13 which is the gadolinium complex of 2-hydroxymethyl-1,4,7,10-tetraazacyclododecane-4,7,10 triacetic acid.

**18.** A diagnostic composition which can be administered to man, characterised in that it comprises at least one complex according to any one of claims 12 to 17.

**19.** A composition according to claim 18, comprising a solution of the complex in an aqueous solvent.

**Claims for the following Contracting State : ES**

**1.** Method for the preparation of ligands of formula:

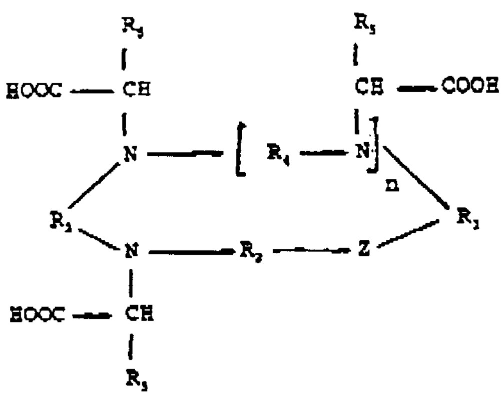

I

in which
$R_1$ represents a radical of formula

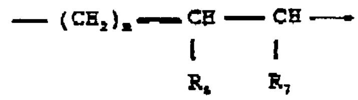

$R_6$ being selected from a $C_1$-$C_{14}$ alkyl group, a $C_1$-$C_4$ hydroxyalkyl group and a $C_1$-$C_4$ polyhydroxyalkyl group and a group having the formula

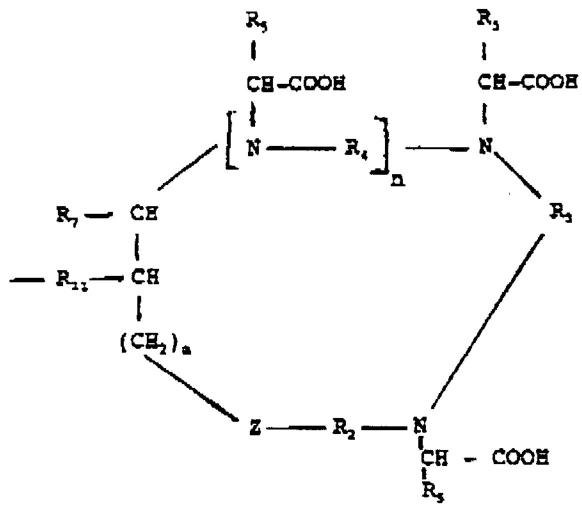

$R_{11}$ being selected from groups A and groups of formula $-(CH_2)_t$ - Y - A - Y - $(CH_2)_t$-
A being selected from $C_1$-$C_8$ alkylene groups, $C_1$-$C_8$ hydroxalkylene and $C_1$-$C_8$ polyhydroxyalkylene groups,
Y being selected from

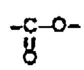

and -O- and t = 1 to 4,
$R_7$ being selected from a hydrogen atom, a $C_1$-$C_{14}$ alkyl group, a $C_1$-$C_4$ hydroxyalkyl group and a $C_1$-$C_4$ polyhydroxyalkyl group,
m = 0 or 1
$R_2$,$R_3$,$R_4$, which may be identical or different, representing a radical of formula

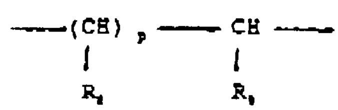

$R_8$ and $R_9$, which may be identical or different, being selected from a hydrogen atom, a $C_1$-$C_{14}$ alkyl group, a $C_1$-$C_4$ hydroxyalkyl group and a $C_1$-$C_4$ polyhydroxyalkyl group,
p = 1 or 2
n = 0,1 or 2 and
$R_5$ is selected from a hydrogen atom, a $C_1$-$C_4$ alkyl group, a $C_1$-$C_4$ hydroxyalkyl group and a $C_1$-$C_4$ polyhydroxyalkyl group, and
Z is selected from a hydrogen atom and a group having the formula

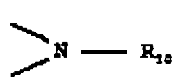

$R_{10}$ being selected from a hydrogen atom, a $C_1$-$C_{14}$ alkyl group, a $C_1$-$C_4$ hydroxyalkyl group, a $C_1$-$C_4$

polyhydroxyalkyl group, a group of formula

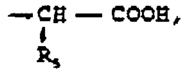

$R_5$ having the meaning above, and a group having the formula

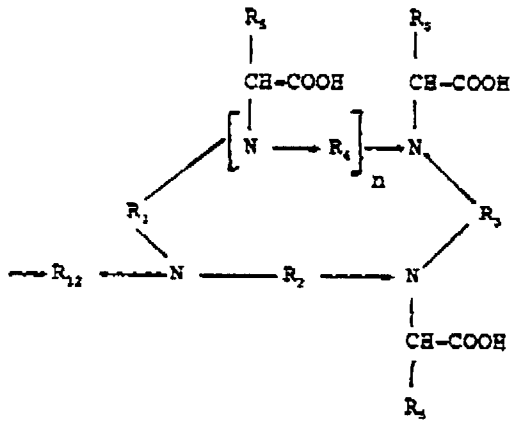

$R_{12}$ being selected from $C_1$-$C_8$ alkylene, $C_1$-$C_8$ hydroxyalkylene and $C_1$-$C_8$ polyhydroxyalkylene groups,
and their salts, characterised in that a compound of formula

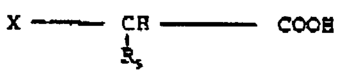 II

in which $R_5$ has the meaning indicated above and X represents a labile group or an aldehyde of formula

$R_5$ — CHO IIa

in which $R_5$ has the meaning given above is reacted in the presence of hydrocyanic acid or cyanide ions, with a cyclic amine of formula

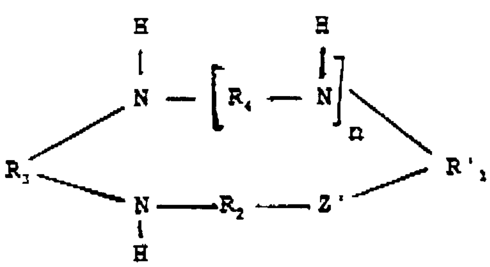 III

in which $R_2$, $R_3$, $R_4$ and n have the meanings given above,
R'$_1$ represents a radical of formula

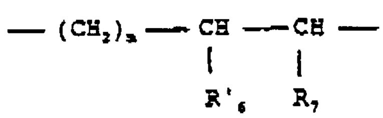

R'$_6$ being selected from a $C_1$-$C_{14}$ alkyl group, a $C_1$-$C_4$ hydroxyalkyl group, a $C_1$-$C_4$ polyhydroxyalkyl

group and a group having the formula

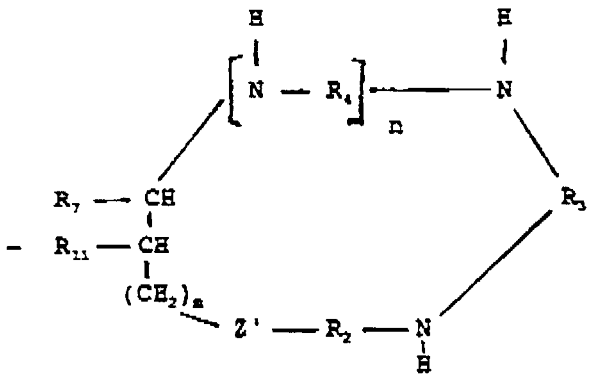

$R_2$, $R_3$, $R_4$, $R_7$, $R_{11}$, m, n having the meanings given above and Z' being selected from an oxygen atom and a group having the formula

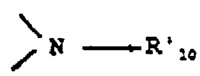

$R'_{10}$ being selected from a hydrogen atom, a $C_1$-$C_{14}$ alkyl group, a $C_1$-$C_4$ hydroxyalkyl group, a $C_1$-$C_4$ polyhydroxyalkyl group and a group having the formula

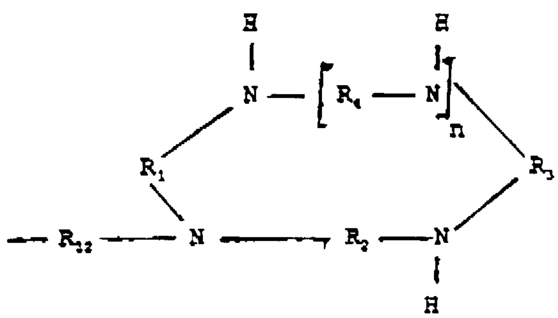

$R_1$, $R_2$, $R_3$, $R_4$, $R_{12}$ and n having the meanings given above.

**2.** A method according to claim 1, characterised in that the compound of formula III is prepared by reacting a polyamine of formula

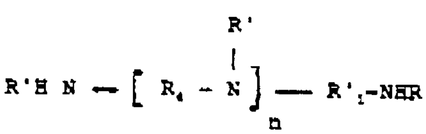 IV

in which $R_1$ and $R_2$ have the meanings given in claim 1 and R' represents a tosyl, mesyl or benzene sulphonyl group
with a compound of formula

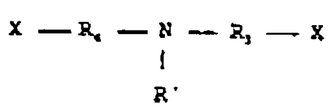 V

in which $R_3$, $R_4$ and R' have the meanings given in claim 1 and X represents a labile group.

**3.** A method according to claim 1, characterised in that the compound of formula III is prepared by reacting a diamine of formula

$R'HN - R'_1 - NH - R'$ X

in which $R'_1$ and R' have the meanings given in claim 1, with a compound of formula

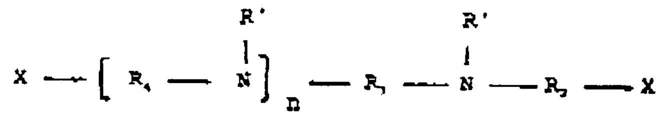

XI

in which n, $R_2$, $R_3$, $R_4$ and R' have the meanings given in claim 1 and X represents a labile group.

**4.** A method for the preparation of complexes formed by ligands of formula I defined in claim 1 with metal ions selected from ions of the lanthanides (atomic numbers 57 to 71), ions of the transition metals (atomic numbers 21 to 29) and the ions of metals of atomic numbers 55, 56, 82, 83, and the salts of these complexes with pharmaceutically acceptable mineral or organic bases or basic amino acids, characterised in that the ligand is reacted with a salt or an oxide of the metals in an aqueous solvent and neutralised if necessary to form a salt.

**5.** A method for the preparation of a diagnostic composition which can be administered to man, characterised in that a complex as defined in claim 4 is made into a pharmaceutically acceptable form.

**Patentansprüche**
**Patentansprüche für folgende Vertragsstaaten : AT, BE, CH, DE, FR, GB, GR, IT, LI, LU, NL, SE**

**1.** Liganden der Formel

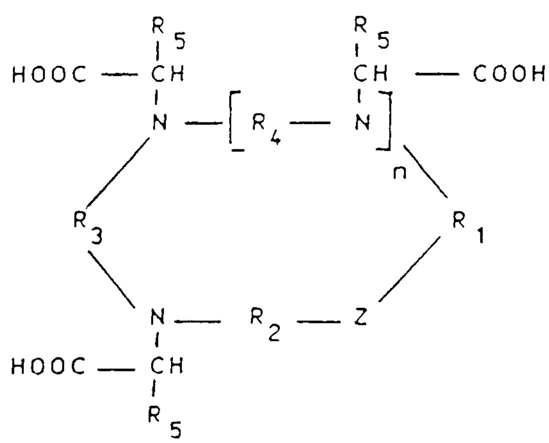

I

in der
$R_1$ eine Gruppe der Formel

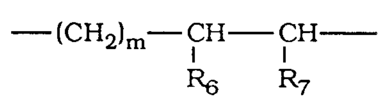

darstellt,
worin $R_6$ aus $C_1$-$C_{14}$-Alkylgruppen, $C_1$-$C_4$-Hydroxyalkylgruppen, $C_1$-$C_4$-Polyhydroxyalkylgruppen und

Gruppen der Formel

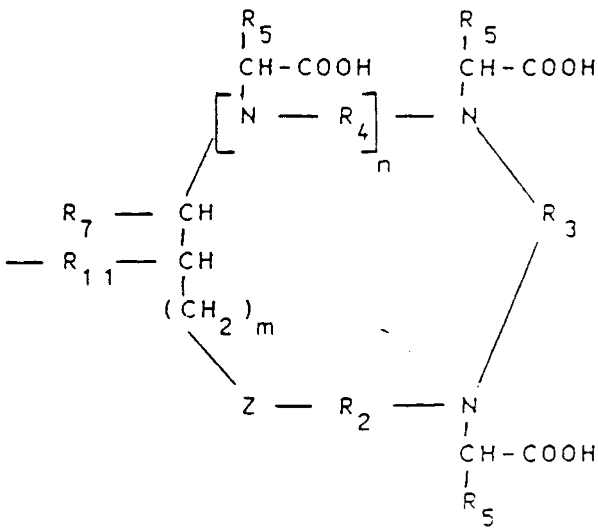

ausgewählt ist,
$R_{11}$ aus Gruppen A und Gruppen der Formel

$-(CH_2)_t - Y - A - Y - (CH_2)_t -$

ausgewählt ist, worin
A aus $C_1$-$C_8$-Alkylengruppen, $C_1$-$C_8$-Hydroxyalkylengruppen und $C_1$-$C_8$-Polyhydroxyalkylengruppen ausgewählt ist,
Y ausgewählt ist aus

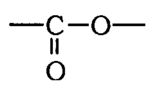

und -O-
und t 1 bis 4 bedeutet,
$R_7$ ausgewählt ist aus Wasserstoffatomen, $C_1$-$C_{14}$-Alkylgruppen, $C_1$-$C_4$-Hydroxyalkylgruppen und $C_1$-$C_4$-Polyhydroxyalkylgruppen,
m 0 oder 1 bedeutet,
$R_2$, $R_3$, $R_4$, die gleichartig oder verschieden sein können, Gruppen der Formel

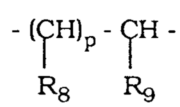

bedeuten,
$R_8$ und $R_9$, die gleichartig oder verschieden sein können, ausgewählt sind aus Wasserstoffatomen, $C_1$-$C_{14}$-Alkylgruppen, $C_1$-$C_4$-Hydroxyalkylgruppen und $C_1$-$C_4$-Polyhydroxyalkylgruppen,
p 1 oder 2 bedeutet,
n 0, 1 oder 2 bedeutet und
$R_5$ ausgewählt ist aus Wasserstoffatomen, $C_1$-$C_4$-Alkylgruppen, $C_1$-$C_4$ Hydroxyalkylgruppen und $C_1$-$C_4$-Polyhydroxyalkylgruppen und
Z ausgewählt ist aus Sauerstoffatomen und Gruppen der Formel

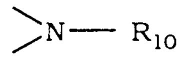

$R_{10}$ ausgewählt ist aus Wasserstoffatomen, $C_1$-$C_{14}$-Alkylgruppen, $C_1$-$C_4$-Hydroxyalkylgruppen, $C_1$-$C_4$-Polyhydroxyalkylgruppen, Gruppen der Formel

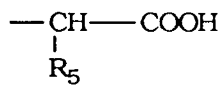

in der $R_5$ die oben angegebenen Bedeutungen besitzt, und Gruppen der Formel

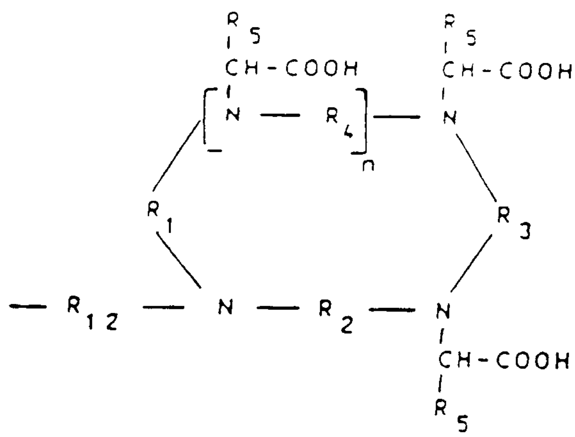

und
$R_{12}$ ausgewählt ist aus $C_1$-$C_8$-Alkylengruppen, $C_1$-$C_8$-Hydroxyalkylengruppen und $C_1$-$C_8$-Polyhydroxyalkylengruppen, sowie deren Salze.

**2.** Liganden der Formel I nach Anspruch 1, worin m den Wert 0 besitzt.

**3.** Verbindung nach Anspruch 1, nämlich 2,6-Dimethyl-1,4,7,10-tetraazacyclododecan-N,N',N",N"'-tetraessigsäure.

**4.** Verbindung nach Anspruch 1, nämlich 2-Hexyl-1,4,7,10-tetraazacyclododecan-N,N',N",N"'-tetraessigsäure.

**5.** Verbindung nach Anspruch 1, nämlich 2-Methyl-1,4,7,10-tetraazacyclododecan-N,N',N",N"'-tetraessigsäure.

**6.** Verbindung nach Anspruch 1, nämlich 2-Hydroxymethyl-1,4,7,10-tetraazacyclododecan-4,7,10-triessigsäure.

**7.** Verbindung nach Anspruch 1, nämlich 2-Hydroxymethyl-1,4,7,10-tetraazacyclododecan-1,4,7,10-tetraessigsäure.

**8.** Verbindung nach Anspruch 1, nämlich 2-(2-Hydroxyethyl)-1,4,7,10-tetraazacyclododecan-1,4,7,10-tetraessigsäure.

**9.** Verfahren zur Herstellung einer Verbindung nach Anspruch 1 oder 2, **dadurch gekennzeichnet,** daß man eine Verbindung der Formel

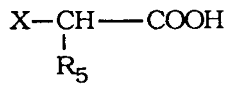 (II)

in der $R_5$ die in Anspruch 1 angegebenen Bedeutungen besitzt und X eine labile Gruppe darstellt, oder einen Aldehyd der Formel

$R_5—CHO$ (IIa)

In der $R_5$ die in Anspruch 1 angegebenen Bedeutungen besitzt, in Gegenwart von Cyanwasserstoffsäure oder Cyanidionen mit einem cyclischen Amin der Formel

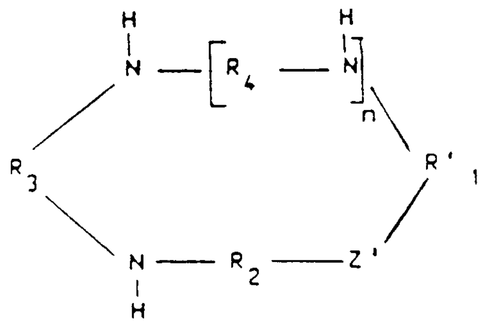 III

in der $R_2$, $R_3$, $R_4$ und n die oben angegebenen Bedeutungen besitzen,
$R'_1$ eine Gruppe der Formel

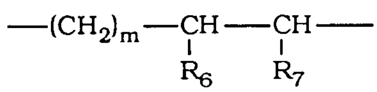

darstellt,
$R'_6$ ausgewählt ist aus $C_1$-$C_{14}$-Alkylgruppen, $C_1$-$C_4$-Hydroxyalkylgruppen, $C_1$-$C_4$-Polyhydroxyalkylgruppen und Gruppen der Formel

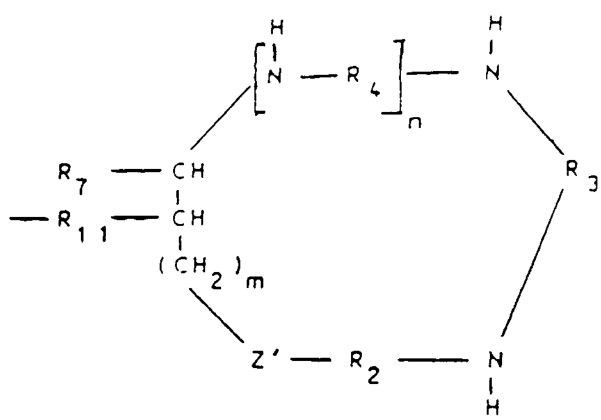

$R_2$, $R_3$, $R_4$, $R_7$, $R_{11}$, m, n die in Anspruch 1 angegebenen Bedeutungen besitzen und Z' ausgewählt ist aus Sauerstoffatomen und Gruppen der Formel

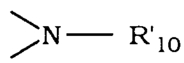

$R'_{10}$ ausgewählt ist aus Wasserstoffatomen, $C_1$-$C_{14}$-Alkylgruppen, $C_1$-$C_4$-Hydroxyalkylgruppen, $C_1$-$C_4$-Polyhydroxyalkylgruppen und Gruppen der Formel

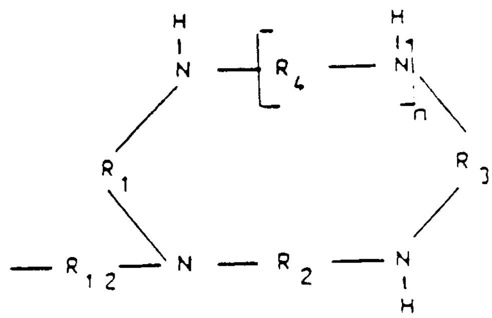

worin $R_1$, $R_2$, $R_3$, $R_4$, $R_{12}$ und n die oben angegebenen Bedeutungen besitzen, umsetzt.

10. Verfahren nach Anspruch 9, **dadurch gekennzeichnet,** daß man die Verbindung der Formel (III) durch Umsetzen eines Polyamins der Formel

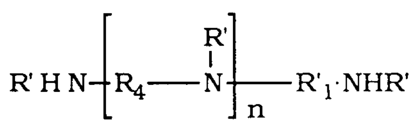

(IV)

in der $R_1$ und $R_2$ die in Anspruch 9 angegebenen Bedeutungen besitzen und R' eine Tosylgruppe, Mesylgruppe oder Benzolsulfonylgruppe darstellt,
mit einer Verbindung der Formel

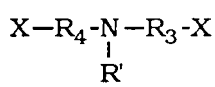

(V)

in der $R_3$, $R_4$ und R' die in Anspruch 9 angegebenen Bedeutungen besitzen und X eine labile Gruppe darstellt, herstellt.

11. Verfahren nach Anspruch 10, **dadurch gekennzeichnet,** daß man die Verbindung der Formel (III) durch Umsetzen eines Diamins der Formel

$R'HN—R'_1—NH—R'$ (X)

in der $R'_1$ und R' die in Anspruch 10 angegebenen Bedeutungen besitzen, mit einer Verbindung der Formel

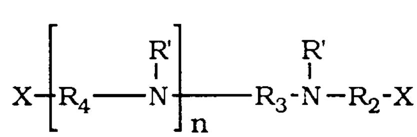

(XI)

in der n, $R_2$, $R_3$, $R_4$ und R' die in Anspruch 10 angegebenen Bedeutungen besitzen und X eine labile Gruppe darstellt, herstellt.

12. Komplexe gebildet durch Liganden der Formel (I) nach einem der Ansprüche 1 bis 8 mit Metallionen ausgewählt aus Ionen der Lanthaniden (Ordnungszahlen 57 bis 71), Ionen von Übergangsmetallen (Ordnungszahlen 21 bis 29) und Ionen von Metallen der Ordnungszahlen 55, 56, 82 und 83, sowie die

Salze dieser Komplexe mit anorganischen oder organischen, pharmazeutisch annehmbaren Basen oder basischen Aminosäuren.

**13.** Komplexe nach Anspruch 12, worin das Metallion aus Ionen von Gadolinium, Europium, Disprosium, Eisen ($Fe^{3+}$) und Mangan ($Mn^{2+}$) ausgewählt wird.

**14.** Komplex nach Anspruch 13, nämlich das Methylglucamin-Salz des Gadolinium-Komplexes der 2,6-Dimethyl-1,4,7,10-tetraazacyclododecan-N,N',N",N"'-tetraessigsäure.

**15.** Komplex nach Anspruch 13, nämlich das Methylglucamin-Salz des Gadolinium-Komplexes der 2-Hexyl-1,4,7,10-tetraazacyclododecan-N,N',N",N"'-tetraessigsäure.

**16.** Komplex nach Anspruch 13, nämlich das Methylglucamin-Salz des Gadolinium-Komplexes der 2'-Methyl-1,4,7,10-tetraazacyclododecan-N,N',N",N"'-tetraessigsäure.

**17.** Komplex nach Anspruch 13, nämlich der Gadolinium-Komplex der 2-Hydroxymethyl-1,4,7,10-tetraazacyclododecan-4,7,10-triessigsäure.

**18.** An Menschen zu verabreichen des diagnostisches Mittel, **dadurch gekennzeichnet,** daß es mindestens einen Komplex nach einem der Ansprüche 12 bis 17 enthält.

**19.** Mittel nach Anspruch 18 in Form einer Lösung des Komplexes in einem wäßrigen Lösungsmittel.

**Patentansprüche für folgenden Vertragsstaat : ES**

**1.** Verfahren zur Herstellung von Liganden der Formel

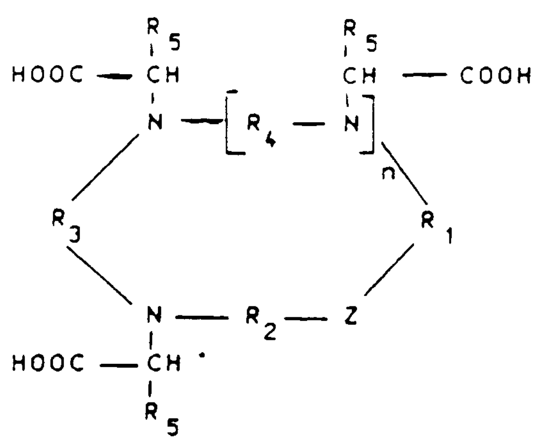

I

in der
$R_1$ eine Gruppe der Formel

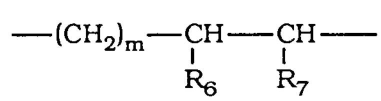

darstellt,
worin $R_6$ aus $C_1$-$C_{14}$-Alkylgruppen, $C_1$-$C_4$-Hydroxyalkylgruppen, $C_1$-$C_4$-Polyhydroxyalkylgruppen und Gruppen der Formel

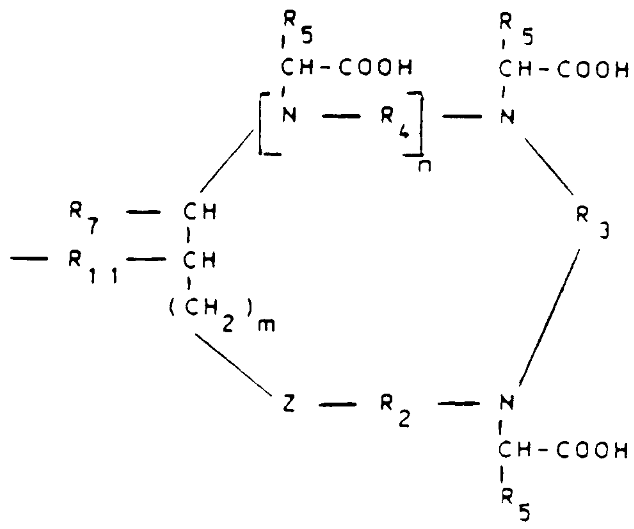

ausgewählt ist,
$R_{11}$ aus Gruppen A und Gruppen der Formel

$$-(CH_2)_t-Y-A-Y-(CH_2)_t-$$

ausgewählt ist, worin
A aus $C_1$-$C_8$-Alkylengruppen, $C_1$-$C_8$-Hydroxyalkylengruppen und $C_1$-$C_8$-Polyhydroxyalkylengruppen ausgewählt ist,
Y ausgewählt ist aus

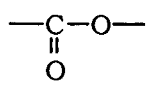

und -O-
und t 1 bis 4 bedeutet,
$R_7$ ausgewählt ist aus Wasserstoffatomen, $C_1$-$C_{14}$-Alkylgruppen, $C_1$-$C_4$-Hydroxyalkylgruppen und $C_1$-$C_4$-Polyhydroxyalkylgruppen,
m 0 oder 1 bedeutet,
$R_2$, $R_3$, $R_4$, die gleichartig oder verschieden sein können, Gruppen der Formel

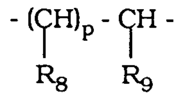

bedeuten,
$R_8$ und $R_9$, die gleichartig oder verschieden sein können, ausgewählt sind aus Wasserstoffatomen, $C_1$-$C_{14}$-Alkylgruppen, $C_1$-$C_4$-Hydroxyalkylgruppen und $C_1$-$C_4$-Polyhydroxyalkylgruppen,
p 1 oder 2 bedeutet,
n 0, 1 oder 2 bedeutet und
$R_5$ ausgewählt ist aus Wasserstoffatomen, $C_1$-$C_4$-Alkylgruppen, $C_1$-$C_4$ Hydroxyalkylgruppen und $C_1$-$C_4$-Polyhydroxyalkylgruppen und
Z ausgewählt ist aus Sauerstoffatomen und Gruppen der Formel

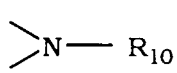

$R_{10}$ ausgewählt ist aus Wasserstoffatomen, $C_1$-$C_{14}$-Alkylgruppen, $C_1$-$C_4$-Hydroxyalkylgruppen, $C_1$-$C_4$-

Polyhydroxyalkylgruppen, Gruppen der Formel

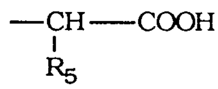

in der $R_5$ die oben angegebenen Bedeutungen besitzt, und Gruppen der Formel

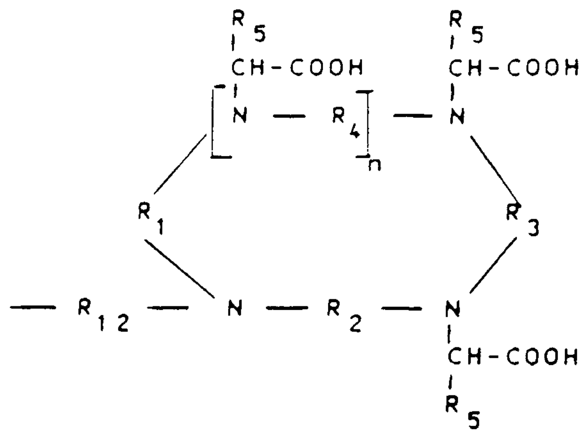

und
$R_{12}$ ausgewählt ist aus $C_1$-$C_8$-Alkylengruppen, $C_1$-$C_8$-Hydroxyalkylengruppen und $C_1$-$C_8$-Polyhydroxyalkylengruppen, sowie deren Salze,
**dadurch gekennzeichnet,** daß man eine Verbindung der Formel

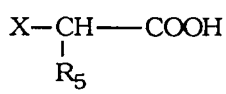

(II)

in der $R_5$ die in Anspruch 1 angegebenen Bedeutungen besitzt und X eine labile Gruppe darstellt, oder einen Aldehyd der Formel

$R_5$—CHO (IIa)

in der $R_5$ die in Anspruch 1 angegebenen Bedeutungen besitzt, in Gegenwart von Cyanwasserstoffsäure oder Cyanidionen mit einem cyclischen Amin der Formel

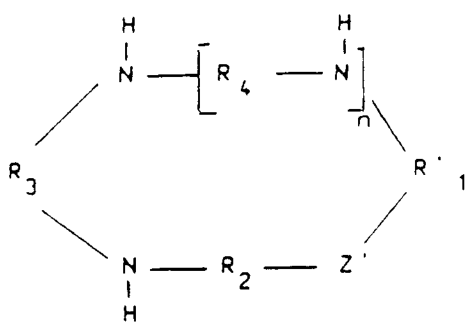

III

in der $R_2$, $R_3$, $R_4$ und n die oben angegebenen Bedeutungen besitzen,
$R'_1$ eine Gruppe der Formel

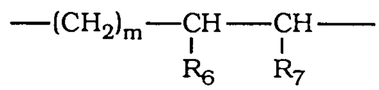

darstellt,
$R'_6$ ausgewählt ist aus $C_1$-$C_{14}$-Alkylgruppen, $C_1$-$C_4$-Hydroxyalkylgruppen, $C_1$-$C_4$-Polyhydroxyalkylgruppen und Gruppen der Formel

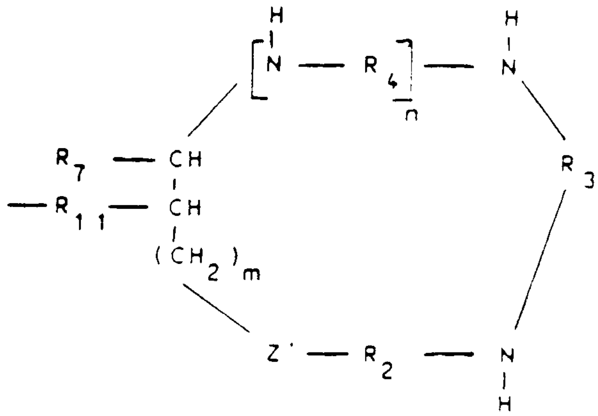

$R_2$, $R_3$, $R_4$, $R_7$, $R_{11}$, m, n die in Anspruch 1 angegebenen Bedeutungen besitzen und Z' ausgewählt ist aus Sauerstoffatomen und Gruppen der Formel

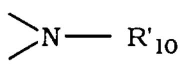

ausgewählt ist,
$R'_{10}$ ausgewählt ist aus Wasserstoffatomen, $C_1$-$C_{14}$-Alkylgruppen, $C_1$-$C_4$-Hydroxyalkylgruppen, $C_1$-$C_4$-Polyhydroxyalkylgruppen und Gruppen der Formel

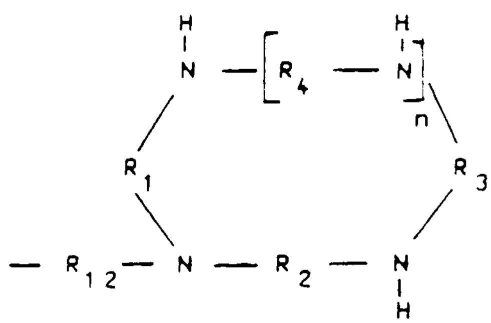

worin $R_1$, $R_2$, $R_3$, $R_4$, $R_{12}$ und n die oben angegebenen Bedeutungen besitzen, umsetzt.

2. Verfahren nach Anspruch 1, **dadurch gekennzeichnet,** daß man die Verbindung der Formel (III) durch Umsetzen eines Polyamins der Formel

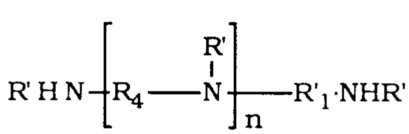 (IV)

in der $R_1$ und $R_2$ die in Anspruch 9 angegebenen Bedeutungen besitzen und R' eine Tosylgruppe, Mesylgruppe oder Benzolsulfonylgruppe darstellt,
mit einer Verbindung der Formel

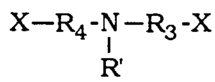 (V)

in der $R_3$, $R_4$ und R' die in Anspruch 9 angegebenen Bedeutungen besitzen und X eine labile Gruppe darstellt, herstellt.

3. Verfahren nach Anspruch 1, **dadurch gekennzeichnet,** daß man die Verbindung der Formel (III) durch Umsetzen eines Diamins der Formel

$R'HN-R'_1-NH-R'$ (X)

in der $R'_1$ und R' die in Anspruch 10 angegebenen Bedeutungen besitzen, mit einer Verbindung der Formel

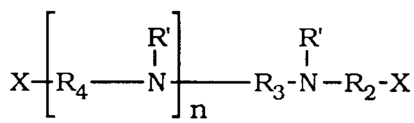 (XI)

in der n, $R_2$, $R_3$, $R_4$ und R' die in Anspruch 10 angegebenen Bedeutungen besitzen und X eine labile Gruppe darstellt, herstellt.

4. Verfahren zur Herstellung von Komplexen, die gebildet sind durch die Liganden der Formel (I), wie es in Anspruch 1 definiert ist, mit Metallionen, die ausgewählt sind aus Ionen der Lanthaniden (Ordnungszahlen 57 bis 71), Ionen von Übergangsmetallen (Ordnungszahlen 21 bis 29) und Ionen von Metallen mit den Ordnungszahlen 55, 56, 82 und 83 sowie der Salze dieser Komplexe mit anorganischen oder organischen, pharmazeutisch annehmbaren Basen oder basischen Aminosäuren, **dadurch gekennzeichnet,** daß man den Liganden mit einem Salz oder einem Oxid der Metalle in einem wäßrigen Lösungsmittel umsetzt und gegebenenfalls zur Bildung eines Salzes neutralisiert.

5. Verfahren zur Herstellung eines an Menschen verabreichbaren diagnostischen Mittels, **dadurch gekennzeichnet,** daß man einen Komplex, wie er in Anspruch 4 definiert ist, in eine pharmazeutisch annehmbare Form bringt.